(19) Europäisches Patentamt
European Patent Office
Office européen des brevets

(11) Numéro de publication: **0 446 141 B1**

# (12) FASCICULE DE BREVET EUROPEEN

(45) Date de publication de fascicule du brevet: **18.01.95**

(51) Int. Cl.[6]: **C07D 487/04**, A61K 31/505, //(C07D487/04,239:00,235:00)

(21) Numéro de dépôt: **91400645.7**

(22) Date de dépôt: **08.03.91**

## (54) Nouveaux dérivés d'imidazo [1,2-C] quinazoline leur procédé de préparation et les compositions pharmaceutiques les renfermant.

(30) Priorité: **09.03.90 FR 9003003**

(43) Date de publication de la demande: **11.09.91 Bulletin 91/37**

(45) Mention de la délivrance du brevet: **18.01.95 Bulletin 95/03**

(84) Etats contractants désignés: **AT BE CH DE DK ES FR GB GR IT LI LU NL SE**

(56) Documents cités:
**EP-A- 0 053 767**
**US-A- 4 129 653**

(73) Titulaire: **ADIR ET COMPAGNIE**
**1 rue Carle Hébert**
**F-92415 Courbevoie Cédex (FR)**

(72) Inventeur: **Bourguignon, Jean-Jacques**
**2 rue du Feldwasser**
**F-67150 Hopsheim (FR)**
Inventeur: **Wermuth, Camille-Georges**
**3 rue de la Côte d'Azur**
**F-67100 Strasbourg (FR)**
Inventeur: **Renaud de la Faverie, Jean-François**
**7 rue des Erables Rocquencourt**
**F-78150 Le Chesnay (FR)**
Inventeur: **Thollon, Catherine**
**4 rue Guénot**
**F-75011 Paris (FR)**
Inventeur: **Lombet, Alain**
**28 av. René Damous**
**F-94500 Champigny (FR)**

EP 0 446 141 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention a pour objet de nouveaux dérivés d'imidazo [1,2-c] quinazoline, leur procédé de préparation et les compositions pharmaceutiques les renfermant.

Elle concerne particulièrement les dérivés d'imidazo [1,2-c] quinazoline de formule générale I :

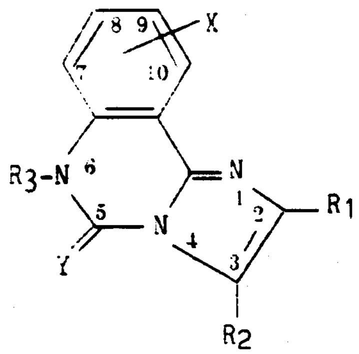

(I)

dans laquelle :

- Y représente un atome d'oxygène ou de soufre ;
- $R_1$ représente :
  a) un radical alkyle ayant de 1 à 6 atomes de carbone en chaine droite ou ramifiée, éventuellement substitué par un radical phényle lui-même éventuellement mono- ou di-substitué par un atome d'halogène ou un radical alkyl ou alkoxy ayant chacun de 1 à 6 atomes de carbone en chaine droite ou ramifiée ;
  b) un radical cycloalkyle mono, bi ou tricyclique renfermant de 3 à 10 atomes de carbone ;
  c) un groupe aromatique choisi parmi le groupe formé de :
  - un radical phényle non substitué et les radicaux phényles mono- et di-substitués par un substituant choisi parmi les atomes d'halogène et les radicaux alkyle et alkoxy ayant chacun de 1 à 6 atomes de carbone en chaine droite et ramifiée, et
  - un radical furyle et un radical thiényle, ou
  d) un radical acyle choisi parmi les radicaux alkoxycarbonyle, aminocarbonyle, N,N-dialkylaminocarbonyle dans lequel le groupe alkyle renferme de 1 à 6 atomes de carbone en chaine droite ou ramifiée, ou un radical benzoyle éventuellement mono- ou di-substitué par un atome d'halogène ou un radical alkyl ou alkoxy ayant chacun de 1 à 6 atomes de carbone en chaine droite ou ramifiée ;
- $R_2$ représente un atome d'hydrogène ou d'halogène ou un radical alkyle contenant de 1 à 6 atomes de carbone éventuellement substitué par un radical amino, alkylamino ou dialkyl amino dans lequel la partie alkyle renferme de 1 à 6 atomes de carbone en chaine droite ou ramifiée ;
- $R_3$ représente :
  a) un atome d'hydrogène,
  b) un radical alkyle ayant de 1 à 6 atomes de carbone en chaine droite ou ramifiée, éventuellement substitué par un radical phényle, lui-même éventuellement mono- ou poly-substitué par un radical alkyle ou alkoxy ayant chacun de 1 à 6 atomes de carbone en chaine droite ou ramifiée, ou :
  c) un radical de formule :

  R-CO-A-

  dans laquelle :
  - A est un radical alkylène en chaîne droite ou ramifiée, ayant de 1 à 6 atomes de carbone, et
  - R est un radical alkoxy ayant de 1 à 6 atomes de carbone ou un radical amino de formule :

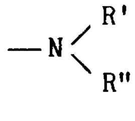

dans laquelle R' et R'', identiques ou différents, représentent chacun un atome d'hydrogène ou un radical alkyle contenant de 1 à 6 atomes de carbone en chaîne droite ou ramifiée éventuellement

subsitué par un radical hydroxy ou alkoxy ayant de 1 à 6 atomes de carbone, ou R' et R" forment ensemble avec l'atome d'azote auquel ils sont liés, un radical hétérocyclique hexagonal choisi parmi les radicaux morpholinyle ou N-méthyl pipérazinyle,

d) un radical phényle, éventuellement substitué par un ou plusieurs radicaux alkyle ou alkoxy ayant chacun de 1 à 6 atomes de carbone en chaine droite ou ramifiée

- X représente un atome d'hydrogène ou d'halogène,

et la phényl-2 pipéridinocarboxylméthyl-6 oxo-5 dihydro-5,6 imidazo[1,2-c] quinazoline.

L'état antérieur de la technique est illustré notamment par :

- Chemical Abstract 77, 126 572 w qui décrit entre autre le dérivé de l'imidazo [1,2-c] quinazolinone de formule :

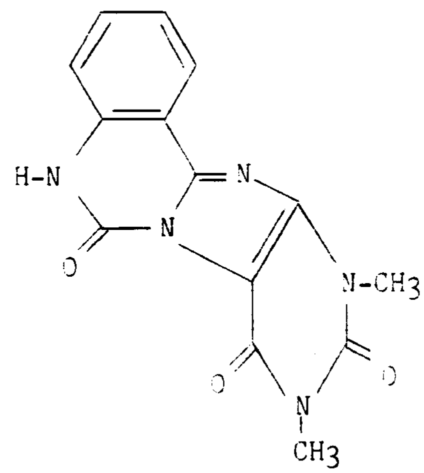

- la demande de brevet européen n° 53.767 qui décrit des dihydro-2,3 imidazo [1,2-c] quinazolinones-5 dont a formule de base est :

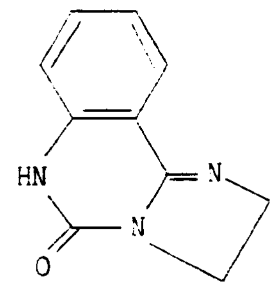

utilisables dans le traitement des maladies circulatoires.

- Journal of Medicinal Chemistry, vol. 18 n° 5, p. 447-453 (1975) qui décrit des dérivés d'imidazo[2,1-b] (10H) quinazolinone-5, dont en particulier le dérivé de formule :

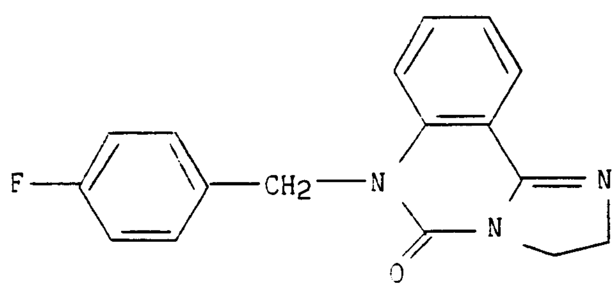

ayant une activité bronchodilatatrice,

- et le brevet US-4 129 653 qui décrit des pyrrolo[1,2-c]quinolines antihypertensives et tonicardiaques.

Aucune de ces références ne décrit ni suggère l'objet de la présente invention, c'est-à-dire les dérivés de formule générale I, ayant le profile pharmacologique ci-après décrit.

La présente invention a également pour objet le procédé de préparation des dérivés de formule générale I, caractérisé en ce que :

- l'on fait réagir sur le dérivé de formule générale II :

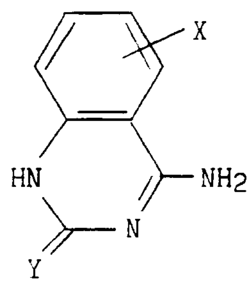

(II)

dans laquelle X et Y ont les significations précédemment définies,
un dérivé halogéné de formule générale III :

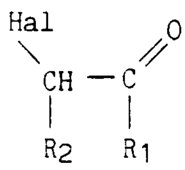

(III)

dans laquelle $R_1$ et $R_2$ ont les significations précédemment définies et Hal représente un atome d'halogène tel que par exemple un atome de brome ou de chlore, pour obtenir le dérivé de formule générale $I_a$ :

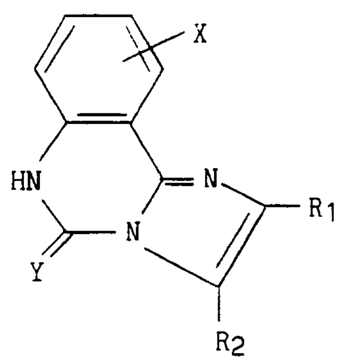

($I_a$)

dans laquelle X, Y, $R_1$ et $R_2$ ont les significations précédemment définies;

- et l'on traite le dérivé $I_a$ ainsi obtenu par une base telle que l'hydrure de sodium, puis par un agent alkylant de formule générale IV :

R'3-Hal (IV)

dans laquelle :
$R'_3$ représente :
a) un radical alkyle ayant de 1 à 5 atomes de carbone en chaîne droite ou ramifiée, éventuellement substitué par un radical phényle lui-même éventuellement mono- ou poly- substitué par un radical alkyle ou alkoxy ayant chacun de 1 à 5 atomes de carbone en chaine droite ou ramifiée, ou
b) un radical de formule :

R-CO-A-

dans laquelle R et A ont les significations précédemment définies ; et Hal représente un atome d'halogène,
pour obtenir le dérivé de formule générale $I_b$ :

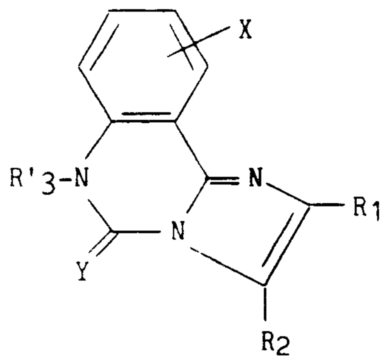

($I_b$)

dans laquelle X, Y, $R_1$, $R_2$ et $R'_3$ ont les significations précédemment définies.

Le procédé ci-dessus décrit est réalisé de façon particulièrement adéquate en effectuant la réaction des dérivés II et III dans un solvant tel que par exemple le diméthylformamide à une température comprise entre 25 et 120 °C, ce qui permet de préparer les dérivés $I_a$ avec des rendements variant de 60 à 85%.

Les dérivés $I_b$ sont préparés avantageusement en traitant le dérivé $I_a$ par l'hydrure de sodium en 30 minutes à température ambiante, en opérant sous atmosphère inerte, dans un solvant dipolaire aprotique comme par exemple le diméthylformamide.

L'ensemble des dérivés de formule générale $I_a$ et $I_b$ forme l'ensemble des dérivés de formule générale I.

Les matières premières de formule générale II ont été préparées à partir d'anthranilonitrile, selon le procédé décrit dans Rec. Trav. Chim. Pays-Bas 79, 443, (1960).

Les matières premières de formule générale III ont été obtenues à partir des méthylcétones correspondantes (qui sont des produits du commerce) par halogénation dans l'éther en présence d'un équivalent d'halogène, à une température variant entre 0 et 25 °C pendant une durée comprise entre 30 et 120 minutes, selon la nature de la cétone de départ. Ainsi préparée, l'α-bromométhylcétone, répondant à la formule III, est utilisée telle quelle après lavage de la phase éthérée avec une solution de bicarbonate de sodium, séchage et évaporation de la phase organique. Dans le cas où $R_1$ représente un radical diméthoxy-3,4 phényle et $R_2$ un atome d'hydrogène, le bromure de diméthoxy-3,4 phénacyle précipite dans le milieu réactionnel.

Dans le cas où $R_1$ représente un radical benzoyle et $R_2$ un atome d'hydrogène, la bromo-3 phényl-1 propanedione-1,2 est préparée selon le procédé décrit dans Helvetica Chimica Acta 29, 1247, (1946).

D'autre part, les dérivés de formule générale $I_b$ dans laquelle $R'_3$ représente un radical de formule : R-CO-A- dans laquelle R est un radical alkoxy de 1 à 6 atomes de carbone et A un radical alkylène en chaîne droite ou ramifiée ayant de 1 à 6 atomes de carbone, peuvent être hydrolysés en milieu HHal/$CH_3COOH$ pour donner les acides correspondants de formule générale V :

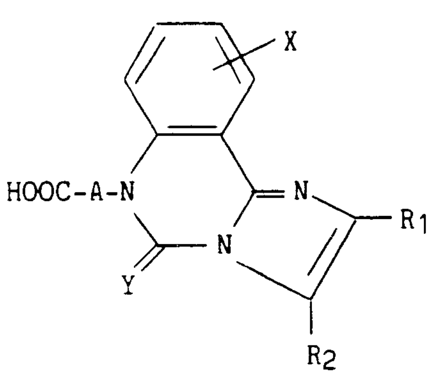

(V)

dans laquelle X, Y, $R_1$, $R_2$ et A ont les significations précédemment définies, lesquels acides, traités par le chlorure d'oxalyle dans le tétrahydrofuranne à 50-60 ° C, donnent les chlorures d'acide correspondants qui, sans étape de purification, sont traités par une amine de formule :

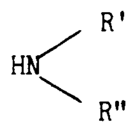

dans laquelle R' et R" ont les significations précédemment définies pour donner les dérivés de formule générale $I_c$ :

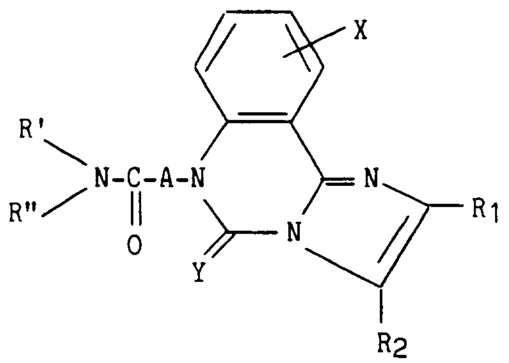

(Ic)

dans laquelle X, Y, $R_1$, $R_2$, A, R' et R" ont les significations précédemment définies ; lesquels dérivés ($I_c$) sont également inclus dans la formule générale I.

Le procédé de préparation des dérivés ($I_c$) ci-dessus décrit, est également inclus dans la présente invention.

Les dérivés de formule générale I donnent des sels avec les acides physiologiquement tolérables -sels qui sont à ce titre, inclus dans la présente invention.

Les dérivés de formule générale I et leurs sels physiologiquement tolérables possèdent des propriétés pharmacologiques et thérapeutiques intéressantes.

En particulier, ce sont des ligands sélectifs et spécifiques des récepteurs aux benzodiazépines périphériques. Par ailleurs, ces composés induisent une relaxation des fibres musculaires lisses situées au niveau des vaisseaux sanguins, du rein, des bronches, etc...

En conséquence, les dérivés de la présente invention s'avèrent utiles pour le traitement de la crise angineuse aiguë, le traitement prophylactique de la crise d'angine de poitrine et de l'ischémie, le traitement de l'hypertension, de l'artériosclérose et d'autres maladies hyperprolifératives et le traitement de l'asthme. Ils sont également utiles dans le traitement de l'anxiété, de la dépression et des troubles d'ordre immunologique.

La présente invention a également pour objet les compositions pharmaceutiques contenant comme principe actif un dérivé de formule générale I ou un de ses sels physiologiquement tolérable, mélangé ou associé à un excipient pharmaceutique approprié comme, par exemple, le glucose, le lactose, l'amidon, le talc, l'éthylcellulose, le stéarate de magnésium ou le beurre de cacao.

Les compositions pharmaceutiques ainsi obtenues se présentent généralement sous forme dosée et peuvent contenir de 1 à 10 mg de principe actif. Elles peuvent revêtir, par exemple, la forme de comprimés, dragées, gélules, suppositoires, solutions injectables ou buvables et être, selon les cas, administrées par voie orale, rectale ou parentérale à la dose de 1 à 10 mg une à deux fois par jour.

Les exemples suivants illustrent la présente invention.

Exemple I :

Ethoxycarbonyl-2 oxo-5 dihydro-5,6 imidazo [1,2-c] quinazoline

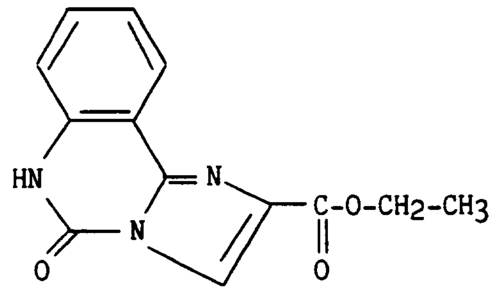

A 1,5 g (0,009 mole) d'amino-4 quinazolinone-2 dans 40 ml de diméthylformamide, on ajoute 1,65 ml (0,013 mole) de bromopyruvate d'éthyle. Après 15 heures d'agitation à température ambiante, on ajoute de l'acétate de sodium jusqu'à obtention d'un pH = 5. L'agitation est maintenue pendant 36 heures en ajustant le pH à 5, puis on ajoute de l'eau et filtre la solution.

Le solide (910 mg) est séché, puis mis dans 40 ml d'éthanol en présence d'un catalyseur, l'acide paratoluène sulfonique. Le milieu réactionnel est porté à reflux pendant 2 heures, puis refroidi et filtré pour séparer les cristaux blancs ainsi formés.

Les eaux mères sont extraites à l'acétate d'éthyle. Après séchage sur $MgSO_4$ et évaporation à sec au rotavapor, le résidu est chauffé à reflux dans l'éthanol en présence d'acide paratoluène sulfonique comme précédemment, puis on laisse refroidir le mélange et filtre les cristaux obtenus.

Après recristallisation dans l'éthanol à 95°, on obtient l'éthoxycarbonyl-2 oxo-5 dihydro-5,6 imidazo [1,2-c] quinazoline, sous forme de cristaux blancs, fondant à 264-266°C.

Rendement : 58%.

CCM ($CH_3COOC_2H_5$-Hexane ; 1-1) : $R_f$ = 0,33

RMN (TFA) δ = 1,5 (t, 3H, J = 7 Hz) ;
4,7 (g, 2H, J = 7 Hz) ;
7,6-8,2 (m, 3H) ;
8,4-8,7 (m, 1H) ; 8,8 (s, 1 H).

Exemple 2 :

Ethoxycarbonyl-2 N,N-diéthylamino-carbonylméthyl-6 oxo-5 dihydro-5,6 imidazo [1,2-c] quinazoline

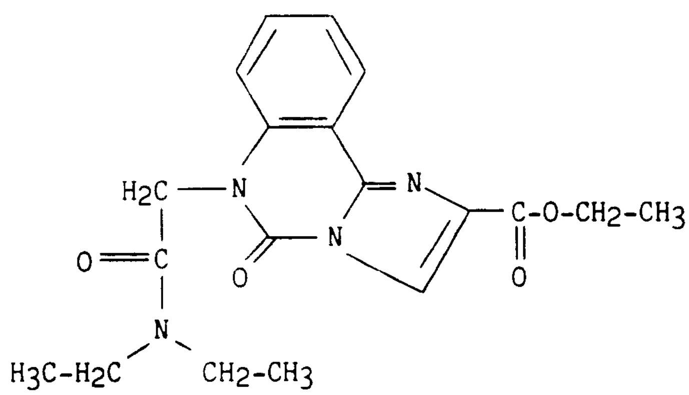

A 0,2 g (0,007 mole) d'éthoxycarbonyl-2 oxo-5 dihydro-5,6 imidazo [1,2-c] quinazoline dissous dans 4 ml de diméthylformamide, on ajoute 0,038 g (1,1 équivalent) d'hydrure de sodium et maintient l'agitation sous atmosphère d'argon pendant 30 minutes. On ajoute 0,21 ml (0,001 mole) de chloro N,N diéthylacétamide. On maintient l'agitation pendant 15 heures, on ajoute 30 ml d'eau et filtre le précipité obtenu.

Après chromatographie sur colonne de silice, en éluant avec $CH_3COOC_2H_5$, on recueille l'éthoxycarbonyl-2 N,N-diéthylaminocarbonylméthyl-6 oxo-5 dihydro-5,6 imidazo [1,2-c] quinazoline, fondant à 170 °C.

Rendement : 50%.

CCM ($CH_3COOC_2H_5$) : Rf : 0,5

RMN ($CDCl_3$) : δ = 1,16 (m, 9H) ; 3,2-3,6 (m, 4H) ; 4,4 (g, 2H, J = 7 Hz) ; 6,2 (s, 1H) ; 7-7,7 (m, 3H) ; 8,4 (s, 1H) ; 8,4-8,6 (m, 1H).

Exemple 3 :

Phényl-2 oxo-5 dihydro-5,6 imidazo [1,2-c] quinazoline

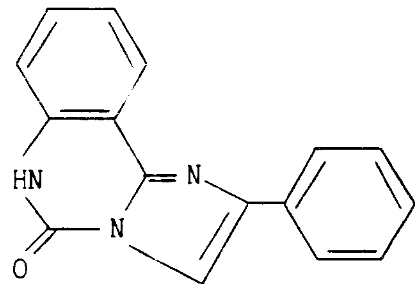

Dans 25 ml de diméthylformamide, on ajoute 1,61 g (0,01 mole) d'amino-4 quinazolinone-2 puis 2,2 g (0,011 mole) de bromure de phénacyle. On chauffe à 120 °C (température extérieure) pendant 3 heures, puis on laisse refroidir et ajoute de l'eau. On filtre le précipité brun formé (1,34 g).

Rendement : 52%.

Le produit ainsi obtenu est suffisamment pur pour être utilisé tel quel pour la suite des synthèses.

Un échantillon a été purifié par chromatographie sur colonne de silice, en éluant avec $CH_3COOC_2H_5$-hexane (1-1), on obtient la phényl-2 oxo-5 dihydro-5,6 imidazo[1,2-c] quinazoline pure, P.F. : 278-280 °C.

CCM ($CH_3COOC_2H_5$-hexane 1-1) : Rf = 0,65

RMN (DMSO) : δ = 3,3 (s large, 1H) ; 7,1-7,7 (m, 6H) ; 7,9-8,3 (m, 3H) ; 8,4 (s, 1H).

L'amino-4 quinazolinone-2 de départ a été préparée selon le procédé de K.W. BREUKINK et P.E. VERKADE - Rec. Trav. Chim. 79, 443-453 (1960).

Exemple 4 :

Amino-carbonyl-2 N,N-diéthylaminocarbonylméthyl-6 oxo-5 dihydro-5,6 imidazo [1,2-c] quinazoline

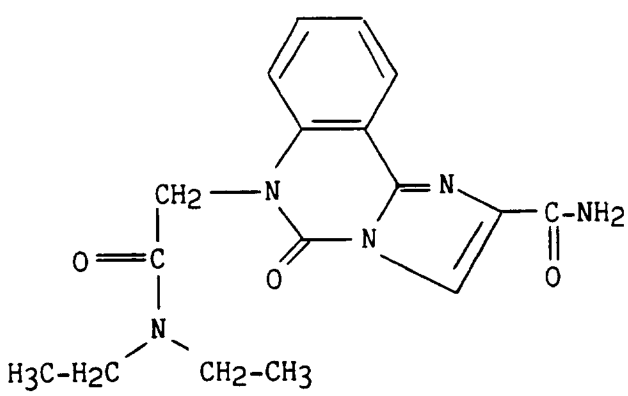

Dans 25 ml de méthanol, on ajoute 0,3 g (0,0008 mole) d'éthoxycarbonyl-2 N,N-diéthylaminocarbonylméthyl-6 oxo-5 dihydro-5,6 imidazo [1,2-c] quinazoline. On sature la solution, refroidie dans un bain de glace, avec de l'ammoniac. On agite pendant 17 heures à température ambiante, on évapore à l'évaporateur rotatif le tiers de la solution et on filtre le précipité formé.

On cristallise le solide dans le méthanol et on obtient 0,14 g d'aminocarbonyl-2 N,N-diéthylaminocarbonylméthyl-6 oxo-5 dihydro-5,6 imidazo [1,2-c] quinazoline, fondant à 248-249 °C.
Rendement : 52%.
CCM (CH3COOC2H5) : Rf = 0,2
RMN (DMSO) : $\delta$ = 1,10 (t, 3H, $\delta$ = 8 Hz) ; 1,35 (t, 3H, $\delta$ = 8 Hz) ; 3,38 (g, 2H, 8 Hz) ; 3,58 (g, 2H, $\delta$ = 8 Hz) ; 5,28 (s, 2H) ; 7,40-7,50 (m, 2H) ; 7,63 (s, 1H) ; 7,69-7,73 (m, 1H) ; 7,67 (s, 1H) ; 8,30 (s, 1H) ; 8,32-8,36 (m, 1H).

Exemple 5 :

Phényl-2 diméthylaminométhyl-3 N,N-diéthylaminocarbonylméthyl-6 oxo-5 dihydro-5,6 imidazo [1,2-c] quinazoline

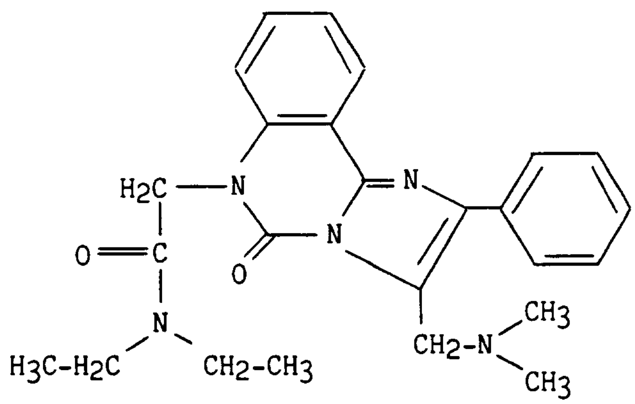

a) A 0,55 g de solution à 35% de diméthylamine refroidie à 5 °C, on ajoute 0,56 g d'acide acétique glacial, puis à cette même température 0,40 g de solution à 35% de formol également refroidie.

On agite doucement et verse dans un ballon contenant 1,04 g ($4.10^{-3}$ mole) de phényl-2 oxo-5 dihydro-5,6 imidazo [1,2-c] quinazoline.

On chauffe jusqu'à dissolution du produit (environ 90 °C) puis abandonne le mélange à température ambiante pendant 24 heures.

On ajoute de la soude 1N jusqu'à pH = 8, extrait à l'acétate d'éthyle, lave à l'eau, sèche sur $Na_2SO_4$ et évapore à sec.

Le produit est purifié par passage sur colonne de silice, en éluant avec l'acétate d'éthyle contenant 5% de triéthylamine. On récupère ainsi 0,74 g ($2,3.10^{-3}$ mole) de phényl-2 diméthylaminométhyl-3 oxo-5 dihydro-5,6 imidazo [1,2-c] quinazoline, sous forme de poudre blanche, fondant à 189-191 °C.
Rendement : 50%.
CCM [$CH_3COOC_2H_5$ à 5% de N $(C_2H_5)_3$] : Rf = 0,3
RMN ($CDCl_3$) : 8,4-7,2 : m, 9H, aromatiques ;
4,2 : s, 2H,

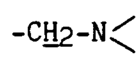 ;

2,3 : s, 6H,

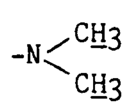 ;

1,25 : s, 1H,

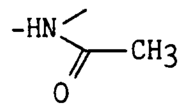

b) Dans un ballon de 50 ml, bien sec, on dissout 0,35 g ($1,1.10^{-3}$ mole) de phényl-2 diméthylaminométhyl-3 oxo-5 dihydro-5,6 imidazo [1,2-c] quinazoline dans 10 ml de diméthylformamide.

On ajoute 0,03 g ($1,21.10^{-3}$ mole) d'hydrure de sodium et laisse sous agitation pendant une demi-heure. On obtient ainsi une solution homogène jaune.

On ajoute 0,3 ml ($2,2.10^{-3}$ mole) de chloro-2 N,N-diéthylacétamide, et maintient l'agitation pendant une nuit à température ambiante. Puis, on ajoute lentement de l'eau au milieu réactionnel et laisse au repos pendant une nuit. On filtre le précipité formé, extrait la phase aqueuse avec $CH_2Cl_2$, sèche sur $Na_2SO_4$ et évapore à sec.

Les deux précipités sont rassemblés et séchés à la pompe à palette. Le produit est purifié par passage sur colonne de silice, en éluant par le système $CH_3COOC_2H_5$-$N(CH_3)_3$, 95-5. On récupère 0,19 g ($0,44.10^{-3}$ mole) de phényl-2 diméthylaminométhyl-3 N,N-diéthylaminocarbonylméthyl-6 oxo-5 dihydro-5,6 imidazo [1,2-c] quinazoline, sous forme de poudre blanche fondant à 192-194 °C.
Rendement : 40%.

| Microanalyse : | | | |
|---|---|---|---|
| | C % | H % | N % |
| Calculé :<br>Trouvé : | 69,58<br>69,44 | 6,77<br>6,91 | 16,23<br>15,90 |

CCM ($CH_3COOC_2H_5$-$N(C_2H_5)_3$, 95-5) : Rf = 0,54
RMN (200 MHZ) ($CDCl_3$) : 9,49-6,99 : m, 9H, aromatiques ;
5,13 : s, 2H,

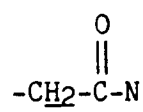 ;

4,12 : s, 2H,

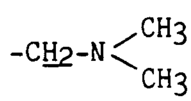 ;

3,56-3,41 : s, 4H, 2 X -C<u>H</u>$_2$-CH3 ;
2,21 : s, 6H,

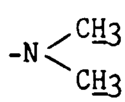 ;

1,40 : t, J = 7, 3H, $-CH_2-C\underline{H}_3$ ;
1,18 : t, J = 7,06, 3H, $-CH_2-C\underline{H}_3$

Exemple 6 :

Benzoyl-2 N,N-diéthylaminocarbonylméthyl-6 oxo-5 dihydro-5,6 imidazo [1,2-c] quinazoline

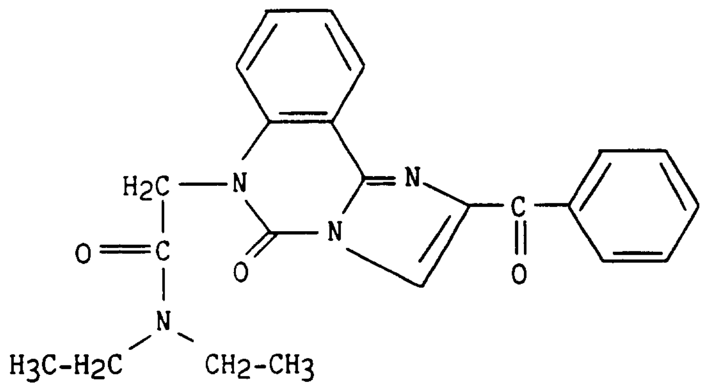

a) Dans un ballon de 50 ml, on ajoute 1 g ($6,2.10^{-3}$ mole) d'amino-4 quinazolinone-2 dans 10 ml de diméthylformamide,1,55 g ($6,8.\ 10^{-3}$ mole) de bromo-3 phényl-1 propanedione-1,2.

Après 15 heures d'agitation à température ambiante, on obtient une solution homogène orange. On y ajoute doucement de l'eau froide, puis on refroidit dans un bain de glace. On filtre le précipité formé, le sèche et le lave à l'éther.

Le produit est purifié par recristallisation dans l'éthanol. On récupère 0,65 0 ($2,25.10^{-3}$ mole) de benzoyl-2 oxo-5 dihydro-5,6 imidazo [1,2-c] quinazoline, sous forme de cristaux jaunes fondant à 260 ° C.
Rendement : 36%.
CCM ($CH_3COOC_2H_5$) : Rf = 0,8
RMN (DMSO) : 8,3 : s, 1H, H-imidazolique ;
8,0-8,2 : m, 3H, aromatiques ;
7,1-7,8 : m, 6H, aromatiques ;
3,5 : s large, 1H,

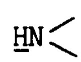

b) Dans un ballon de 50 ml bien sec, on dissout 0,53 g ($1,82.10^{-3}$ mole) de benzoyl-2 oxo-5 dihydro-5,6 imidazo [1,2-c] quinazoline dans 10 ml de diméthylformamide.

On ajoute 0,05 g ($2.10^{-3}$ mole) d'hydrure de sodium. On maintient l'agitation à température ambiante pendant une demi-heure ; on obtient ainsi une solution homogène noire.

On y ajoute 0,60 ml ($3,7.10^{-3}$ mole) de chloro-2 diéthylacétamide et maintient l'agitation pendant une nuit à température ambiante. On ajoute lentement de l'eau au mélange réactionnel. On filtre à la trompe à eau et sèche le produit à la pompe à palette.

Après recristallisation dans le méthanol, on récupère 0,47 g ($1,2.10^{-3}$ mole) de benzoyl-2 N,N-diéthylaminocarbonylméthyl-6 oxo-5 dihydro-5,6 imidazo [1,2-c] quinazoline, sous forme de cristaux jaune pâle fondant à 217-218 ° C.
Rendement : 65%.

| Microanalyse : | | | |
|---|---|---|---|
| | C % | H % | N % |
| Calculé : | 68,64 | 5,51 | 13,92 |
| Trouvé : | 68,76 | 5,44 | 14,19 |

CCM ($CH_3COOC_2H_5$-Hexane, 1-1) : Rf = 0,35
RMN (200 MHz) ($CDCl_3$) : 8,57-7,07 : m, 9H, aromatiques ;

8,37 : s, 1H, H-imidazo ;
5,14 : s, 2H,

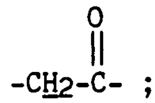

3,58-3,41 : m, 4H, 2 X $-CH_2-CH_3$ ;
1,41 : t, J = 7,12, 3H, $-CH_2-CH_3$ ;
1,18 : t, J = 7,15, 3H, $-CH_2-CH_3$.

Exemple 7 :

Phényl-2 bromo-3 N,N-diéthylaminocarbonylméthyl-6 oxo-5 dihydro-5,6 imidazo [1,2-c] quinazoline

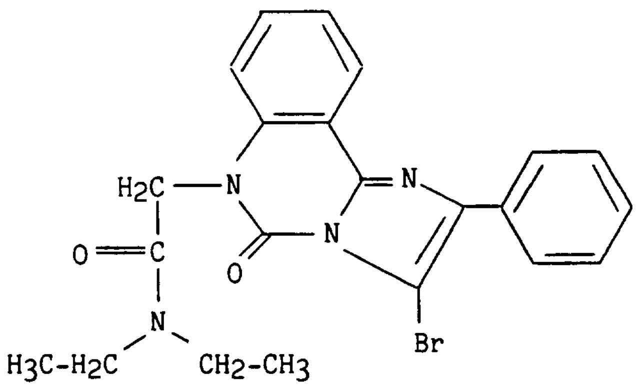

a) Un mélange équimolaire (2.10-3 mole) de phényl-2 oxo-5 dihydro-5,6 imidazo[1,2-c] quinazoline et de N-bromosuccinimide est mis à reflux pendant 16 heures en milieu chloroformique.

Après vérification de la fin de la réaction sur plaque de silice, le chloroforme est évaporé, puis le produit est chromatographié sur colonne de silice (éluant : $CH_3COOC_2H_5$-Hexane, 1-1). On récupère ainsi 0,44 g ($1,3.10^{-3}$ mole) de phényl-2 bromo-3 oxo-5 dihydro-5,6 imidazo [1,2-c] quinazoline, sous forme de poudre blanche, fondant à 235-237 °C.

Rendement : 65%.

CCM ($CH_3COOC_2H_5$-Hexane, 1-1) : Rf = 0,5

RMN (DMSO) : disparition du singulier du H imidazolique.

b) La phényl-2 bromo-3 N,N-diéthylaminocarbonylméthyl-6 oxo-5 dihydro-5,6 imidazo [1,2-c] quinazoline est synthétisée à partir de la phényl-2 bromo-3 oxo-5 dihydro-5,6 imidazo [1,2-c] quinazoline préparée ci-dessus et de la chloro-2 N,N diéthylacétamide selon la méthode décrite dans l'exemple 6 b).

Rendement : 50%.

P.F. : 245-247 °C.

| Microanalyse : | | | |
|---|---|---|---|
| | C % | H % | N % |
| Calculé : | 58,28 | 4,67 | 12,34 |
| Trouve : | 58,32 | 4,64 | 12,20 |

CCM ($CH_3COOC_2H_5$-Hexane, 1-1) : Rf = 0,4

RMN ($CDCl_3$) : 8,45-6,97 : m, 9H, aromatiques ;
5,03 : s, 2H,

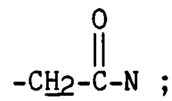

3,43-3,38 : m, 4H, 2 X $-\underline{CH_2}-CH_3$ ;
1,36-1,26 : m, 3H, $-CH2-\underline{CH_3}$ ;
1,16-1,09 : m, 3H, $-CH_2-\underline{CH_3}$.

<u>Exemple 8 - 33</u> :

En opérant, selon les significations des substituants $R_1$, $R_2$ ou $R_3$, comme décrit dans les exemples précédents, ont été préparés les dérivés objets des exemples suivants :

8) Ethoxycarbonyl-2 méthyl-6 oxo-5 dihydro-5,6 imidazo [1,2-c] quinazoline, P.F. :215-216 ° C.
9) Benzoyl-2 oxo-5 dihydro-5,6 imidazo [1,2-c] quinazoline, P.F. : 260 ° C.
10) Ethoxycarbonyl-2 benzyl-6 oxo-5 dihydro-5, 6 imidazo (1,2-c] quinazoline, P.F. : 167-168 ° C.
11) Phényl-2 N,N-diéthylaminocarbonylméthyl-6 oxo-5 dihydro-5,6 imidazo [1,2-c] quinazoline, P.F. : 183-185 ° C.
12) Phényl-2 N-méthylpipérazinocarbonylméthyl-6 oxo-5 dihydro-5,6 imidazo [1,2-c] quinazoline, P.F. du chlorhydrate correspondant : 280-281 ° C.
13) Phényl-2 éthoxycarbonylméthyl-6 oxo-5 dihydro-5,6 imidazo [1,2-c] quinazoline, P.F. : 229 ° C.
14) Phényl-2 N-isopropylaminocarbonylméthyl-6 oxo-5 dihydro-5,6 imidazo [1,2-c] quinazoline, P.F. : 274 ° C.
15) p. Chlorophényl-2 N,N-diéthylaminocarbonylméthyl-6 oxo-5 dihydro-5,6 imidazo [1,2-c] quinazoline, P.F. : 252 ° C.
16) Phényl-2 morpholinocarbonylméthyl-6 oxo-5 dihydro-5,6 imidazo [1,2-c] quinazoline, P.F. : 273-274 ° C.
17) p. Méthoxyphényl-2 N,N-diéthyllaminocarbonylméthyl-6 oxo-5 dihydro-5,6 imidazo [1,2-c] quinazoline, P.F. : 210-212 ° C.
18) p. Méthylphényl-2 N,N-diéthylaminocarbonylméthyl-6 oxo-5 dihydro-5,6 imidazo [1,2-c] quinazoline, P.F. : 233-234 ° C.
19) m. Chlorophényl-2 N,N-diéthylaminocarbonylméthyl-6 oxo-5 dihydro-5,6 imidazo [1,2-c] quinazoline, P.F. : 224-225 ° C.
20) Phényl-2 méthyl-3 N,N-diéthylaminocarbonylméthyl-6 oxo-5 dihydro-5,6 imidazo [1,2-c] quinazoline, P.F. : 192 ° C.
21) (Furyl-2)-2 N,N-diéthylaminocarbonylméthyl-6 oxo-5 dihydro-5,6 imidazo [1,2-c] quinazoline, P.F. : 194 ° C.
22) (Thiényl-3)-2 N,N-diéthylaminocarbonylméthyl-6 oxo-5 dihydro-5,6 imidazo [1,2-c] quinazoline, P.F. :227 ° C.
23) (Diméthoxy-3,4 phényl)-2 N,N-diéthylaminocarbonylméthyl-6 oxo-5 dihydro-5,6 imidazo [1,2-c] quinazoline, P.F. : 204 ° C.
24) Phényl-2 N-isobutylaminocarbonylméthyl-6 oxo-5 dihydro-5,6 imidazo [1,2-c] quinazoline, P.F. : 285 ° C.
25) Phényl-2 éthyl-3 N,N-diéthylaminocarbonylméthyl-6 oxo-5 dihydro-5,6 imidazo [1,2-c] quinazoline, P.F. : 180-181 ° C.
26) Phényl-2 N,N-diéthylaminocarbonylpropyl-6 oxo-5 dihydro-5,6 imidazo [1,2-c] quinazoline, P.F. : 149 ° C.
27) Phényl-2 éthoxycarbonylpropyl-6 oxo-5 dihydro-5,6 imidazo [1,2-c] quinazoline, P.F. : 126 ° C.
28) Phényl-2 N,N-dipropylaminocarbonylméthyl-6 oxo-5 dihydro-5,6 imidazo [1,2-c] quinazoline, P.F. : 189 ° C.
29) Phényl-2 méthyl-6 oxo-5 dihydro-5,6 imidazo [1,2-c] quinazoline, P.F. : 222-225 ° C.
30) o. Chlorophényl-2 N,N-diéthylaminocarbonylméthyl-6 oxo-5 dihydro-5,6 imidazo [1,2-c] quinazoline, P.F. : 188 ° C.
31) Phényl-2 N,N-diéthylaminocarbonylméthyl-6 chloro-10 oxo-5 dihydro-5,6 imidazo [1,2-c] quinazoline, P.F. : 219-220 ° C.
32) m. Chlorophényl-2 méthyl-3 N,N-dipropylaminocarbonylméthyl-6 oxo-5 dihydro-5,6 imidazo [1,2-c] quinazoline, P.F. : 199-201 ° C.

33) Tertiobutyl-2 N,N-diéthylaminocarbonylméthyl-6 oxo-5 dihydro-5,6 imidazo [1,2-c] quinazoline, P.F. : 179 ° C.

34) Adamantan-2-yl N,N-diéthylacétamido-6 oxo-5 dihydro-5,6 imidazo [1,2-c] quinazoline, P.F. : 260-261 ° C.

35) Phényl-2 benzoylméthyl-6 oxo-5 dihydro-5,6 imidazo [1,2-c] quinazoline, P.F. : 273-275 ° C.

36) (3,4-diméthoxyphényl)-2 benzyl-6 oxo-5 dihydro-5,6 imidazo [1,2-c] quinazoline, P.F. : 210-212 ° C.

37) Phényl-2 pipéridinocarboxylméthyl-6 oxo-5 dihydro-5,6 imidazo [1,2-c] quinazoline, P.F. : 245-246 ° C.

38) Phényl-2 N-phénylacétamido-6 oxo-5 dihydro-5,6 imidazo [1,2-c] quinazoline, P.F. : 292-294 ° C.

39) Phényl-2 (N-méthyl N-phénylacétamido)-6 oxo-5 dihydro-5,6 imidazo [1,2-c] quinazoline, P.F. : 240-242 ° C.

40) Tertiobutyl-2 N-N-dipropylacétamido-6 oxo-5 dihydro-5,6 imidazo [1,2-c] quinazoline, P.F. : 145-146 ° C.

41) m-chlorophényl-2 (N-méthyl N-phénylacétamido)-6 oxo-5 dihydro-5,6 imidazo [1,2-c] quinazoline, P.F. : 237-238 ° C.

42) m-chlorophényl-2 bromo-3 (N-méthyl N-phénylacétamido)-6 oxo-5 dihydro-5,6 imidazo [1,2-c] quinazoline, P.F. : 245-247 ° C.

43) phényl-2 (N,N diéthyl méthylacétamido)-6 oxo-5 dihydro-5,6 imidazo [1,2-c] quinazoline, P.F. : 170 ° C.

44) phényl-2 (N,N di(méthoxy-2 éthyl)acétamido)-6 oxo-5 dihydro-5,6 imidazo [1,2-c] quinazoline, P.F. : 203-204 ° C.

Exemple 45 : **ETUDE PHARMACOLOGIOUE**

L'action des dérivés de la présente invention a été démontrée à partir de résultats provenant d'expériences biochimiques et physiologiques. La spécificité d'action ainsi que la sélectivité des composés étudiés à l'égard des récepteurs dits périphériques des benzodiazépines ont été démontrées. L'association des composés étudiés avec leur récepteur s'est révélée être sans effet pharmacologique notable sur le coeur et le système nerveux central et hautement sélectif du muscle lisse coronaire. Les composés étudiés induisent une relaxation dose-dépendante du muscle lisse pré-contracté par addition de $PGF_{2\alpha}$. La relaxation induite par les composés de la présente invention a été évaluée de façon comparative à quelques produits de référence : l'efloxate, le dipyridamole, le RO5-4864, le PK 11195 et le diazepam.

**A) Matériel et méthode** : Tous les produits étudiés ont été mis en solution dans du DMSO à la concentration de $10^{-2}$M, puis dilués aux concentrations voulues dans les milieux expérimentaux. Toutes les valeurs $ED_{20}$, $ED_{50}$ et $K_{0.5}$ ont été corrigées des valeurs obtenues avec le DMSO dans les mêmes conditions de dilution.

1) Etude de liaison : Les études de liaison ont été effectuées sur des fractions microsomales provenant de système nerveux central de rat, [KRUEGER B.K., RATZLAFF R.W., STRICHARTZ G.R. and BLAUSTEIN M.P., Saxitoxin binding to synaptosomes, membranes and solubilized binding sites from rat brain. J. Membrane Biol., 50, 287-310, (1979)] pour les études de déplacement du [$^3$H] RO 151788, fixé de façon spécifique aux récepteurs centraux des benzodiazépines [MÖHLER H., BURKARD W.P., KELLER H.H., RICHARDS J.G. and HAEFELY W. Benzodiazepines antagonist RO-151788 : binding characteristics and interaction with drug induced changes in dopamine turnover and cerebellar GMP levels. J. Neurochem., 37, 714-722, (1981)] et sur des fractions microsomales provenant de coeur de rat [LOMBET A., RENAUD J.F., CHICHEPORTICHE R. and LAZDUNSKI M. A cardiac tetrodotoxin binding component : biochemical identification, characterization and properties. Biochemistry, 20, 1279-1285, (1981)] pour les études de déplacement du [$^3$H] PK 11195 et du RO5-4864 fixés de façon spécifique aux récepteurs périphériques dits des benzodiazépines [LE FUR G., VAUCHER N., PERRIER M.L., FLAMIER A., BENAVIDES J., RENAULT C., DUBROEUCQ M.C., GUEREMY C. and UZAN A. Differentiation between two ligands for peripheral benzodiazepine binding site [$^3$H] RO5-4864 and [$^3$H] PK 11195 by thermodynamic studies. Life Soi., 33, 449-457, (1983)]. Les expériences de déplacement des différents ligands radiomarqués par les molécules de référence et les composés de la présente invention sont conduites dans les conditions standards de liaison ci-dessus mentionnées pour chacun d'entre-eux. Dans tous les cas, après le temps requis d'association, la radioactivité liée est séparée de la composante libre par filtration sous vide sur filtre GF/C. La radioactivité restante sur les filtres est alors déterminée par comptage en scintillation liquide.

2) Flux ioniques

Les influx de $Na^+$ passant par le canal $Na^+$ dont le fonctionnement est dépendant du potentiel de membrane, par l'échangeur $Na^+/H^+$, par le cotransporteur $Na^+/K^+/2Cl^-$ et par la pompe à $Na^+$, ont été

déterminés selon les protocoles précédemment publiés [FRELIN C., VIGNE P. and LAZDUNSKI M. The role of the $Na^+/H^+$ exchange in the regulation of the internal pH in cultured cardiac cells. Eur. J. Biochem., 149, 1-4, (1985) - RENAUD J.F., Internal pH, $Na^+$ and $Ca^{2+}$ regulation by trimetazidine during cardiac cell acidosis. Cardiovasc. Drugs Ther, 1, 677-686, (1988) - FRELIN C., CHASSANDE O. and LAZDUNSKI M. Biochemical characterization of the $Na^+/K^+/2Cl^-$ co-transport in chick cardiac cells. Biochem. Biophys. Res. Commun., 134, 326-331, (1986) et KAZAZOGLOU T., RENAUD J.F., ROSSI B. and LAZDUNSKI M. Two classes of ouabain receptors in chick ventricular cardiac cells and their relation to ($Na^+$, $K^+$) ATPase inhibition, intracellular $Na^+$ accumulation, $Ca^{2+}$ influx and cardiotonic effect. J. Biol. Chem., 258, 12163-12170, (1983)] à partir de cellules cardiaques. Les influx de $Ca^{2+}$ passant par le canal $Ca^{2+}$ lent et par l'échangeur $Na^+/Ca^{2+}$ ont été déterminés à partir d'une lignée de cellules aortiques de rat, $A_7r_5$ [GALIZZI J.P., QAR J., FOSSET M., VAN RENTERGHEM C. and LAZDUNSKI M. Regulation of calcium channels in aortic muscle cells by protein kinase C activators (diacylglycerol and phorbol esters) and by peptides (vasopressin and bombesin) that stimulate phosphoinositide breakdown. J. Biol. Chem., 262, 6947-6950, (1987)] et de cellules cardiaques [KAZAZOGLOU T., RENAUD J.F., ROSSI B. and LAZDUNSKI M. Two classes of ouabain receptors in chick ventricular cardiac cells and their relation to ($Na^+$, $K^+$) ATPase inhibition, intracellular $Na^+$ accumulation, $Ca^{2+}$ influx and cardiotonic effect. J. Biol. Chem., 258, 12163-12170, (1983)] respectivement.

3) Relaxation de la coronaire de porc

Les coronaires sont prélevées sur le coeur de porc (micro-porc Yucatan), anesthésiés au stresnil et pentobarbital. Les anneaux proximaux de la coronaire sont placés dans une cuve d'organe remplie de Ringer à 37 °C. Les anneaux sont tendus progressivement jusqu'à une tension de base d'environ 6 g et contractés par addition de $PGF_{2\alpha}$ à la concentration de $4.10^{-6}$ M. Les potentialités à relaxer l'artère coronaire des produits de la présente invention sont alors testées.

**B) Résultats**

1) Etude de liaison

Ce travail a été réalisé par déplacement du RO 151788 fixé de façon spécifique sur les récepteurs benzodiazépines centraux (système nerveux central de rat), ainsi que par le déplacement du PK 11195 et RO5-4864 fixés de façon spécifique sur les récepteurs benzodiazépines périphériques provenant de coeur de rat. Ces études ont permis de sélectionner des composés présentant une excellente sélectivité pour les récepteurs périphériques avec des affinités comprises entre $10^{-7}$ M et $10^{-8}$ M (tableau I). Une bonne corrélation est trouvée entre les valeurs obtenues pour le déplacement de l'agoniste et de l'antagoniste fixés de façon spécifique au site récepteur périphérique par les dérivés de la présente invention (Figure 1). Par ailleurs, ces composés ont été testés pour d'éventuelles interactions avec les récepteurs d'adénosine ($A_1$, $A_2$), alpha ($\alpha_1$, $\alpha_2$) et beta adrénergique, dopamine ($D_1$, $D_2$), sérotonine ($5HT_{1A}$, $5HT_{1B}$, $5HT_2$) et GABA. Aucune interaction significative n'a pu être relevée pour l'ensemble des composés examinés.

2) Flux ioniques et transport des ions

Ces études ont été réalisées sur des cellules cardiaques et musculaires lisses dans les conditions de la culture in vitro. Tous les composés se sont révélés inactifs sur les systèmes testés ; canal $Na^+$, canal $Ca^{2+}$, échangeur $Na^+/H^+$, échangeur $Na^+/Ca^{2+}$, cotransporteur $Na^+/K^+/2Cl^-$ et la pompe à $Na^+$.

3) Relaxation de la coronaire de porc

L'ensemble des composés ont été testés pour leur capacité à relaxer la coronaire de porc préalablement contractée par la $PGF_{2\alpha}$. Les résultats obtenus (Tableau II) montrent qu'il existe une bonne corrélation entre le niveau de relaxation obtenu ($ED_{50}$) et les valeurs de demi-effet ($K_{0.5}$) obtenues après déplacement du [$^3$H] PK 11195 et du [$^3$H] RO5-4864 fixé de façon spécifique au niveau du récepteur périphérique.

Le niveau de relaxation obtenu ainsi que la valeur de demi-effet ($K_{0.5}$) obtenue par déplacement montrent que sont particulièrement intéressants les composés objet des exemples 10, 11, 17, 28 et 32.

TABLEAU I

Spécificité d'action des composés de l'invention vis-à-vis des récepteurs périphériques marqués par le PK 11195 et le RO5-4864 et des récepteurs centraux marqués par le RO 151788

| | Récepteurs périphériques | | Récepteurs centraux |
|---|---|---|---|
| Produits testés | [$^3$H] PK 11195 $K_{0.5}$ (M) | [$^3$H] RO 54864 $K_{0.5}$ (M) | [$^3$H] RO 151788 $K_{0.5}$ (M) |
| Exemple 1 | $> 10^{-4}$ | 6,1 $10^{-5}$ | $10^{-6}$ |
| Exemple 2 | 5,5 $10^{-7}$ | / | 7 $10^{-5}$ |
| Exemple 3 | 4,5 $10^{-6}$ | $\geqslant 10^{-4}$ | 3,5 $10^{-7}$ |
| Exemple 4 | 8 $10^{-6}$ | 6 $10^{-6}$ | $> 10^{-4}$ |
| Exemple 5 | 2,2 $10^{-6}$ | 4,5 $10^{-6}$ | 1,8 $10^{-4}$ |
| Exemple 6 | 2,2 $10^{-7}$ | 2 $10^{-7}$ | 4,6 $10^{-5}$ |
| Exemple 8 | $\geqslant 10^{-4}$ | 3 $10^{-5}$ | 1,3 $10^{-4}$ |
| Exemple 9 | 2,8 $10^{-5}$ | 1,3 $10^{-5}$ | 1,5 $10^{-7}$ |
| Exemple 10 | $\sim 10^{-4}$ | 8,8 $10^{-6}$ | 4 $10^{-6}$ |
| Exemple 11 | 4 $10^{-7}$ | 8 $10^{-8}$ | $> 10^{-4}$ |
| Exemple 12 | 8 $10^{-5}$ | 4 $10^{-5}$ | $\geqslant 10^{-4}$ |
| Exemple 13 | $10^{-4}$ | $\geqslant 10^{-4}$ | $> 10^{-4}$ |
| Exemple 14 | $\geqslant 10^{-4}$ | $\geqslant 10^{-4}$ | $> 10^{-4}$ |
| Exemple 15 | 1,5 $10^{-6}$ | $\sim 10^{-4}$ | $> 10^{-4}$ |
| Exemple 16 | $> 10^{-4}$ | $> 10^{-4}$ | $> 10^{-4}$ |
| Exemple 17 | 1,4 $10^{-7}$ | 1,2 $10^{-7}$ | $10^{-4}$ |
| Exemple 18 | 2,1 $10^{-7}$ | 7 $10^{-8}$ | $10^{-4}$ |
| Exemple 19 | 5,8 $10^{-8}$ | 5 $10^{-8}$ | $> 10^{-4}$ |
| Exemple 20 | 2,9 $10^{-8}$ | 8,6 $10^{-9}$ | 6 $10^{-5}$ |
| Exemple 21 | 2,6 $10^{-7}$ | 5,8 $10^{-7}$ | $> 10^{-4}$ |
| Exemple 22 | 2 $10^{-7}$ | 1,3 $10^{-7}$ | $> 10^{-4}$ |
| Exemple 23 | 1,5 $10^{-7}$ | 5 $10^{-7}$ | $> 10^{-4}$ |
| Exemple 24 | $\geqslant 10^{-4}$ | $\geqslant 10^{-4}$ | $> 10^{-4}$ |
| Exemple 25 | 3,2 $10^{-8}$ | 5 $10^{-8}$ | 5 $10^{-5}$ |
| Exemple 26 | $10^{-5}$ | 2,3 $10^{-6}$ | $10^{-4}$ |
| Exemple 27 | $\geqslant 10^{-4}$ | 6 $10^{-5}$ | $10^{-4}$ |
| Exemple 28 | 1,1 $10^{-8}$ | 1,2 $10^{-8}$ | $10^{-4}$ |
| Exemple 29 | $\geqslant 10^{-4}$ | $> 10^{-4}$ | 1,5 $10^{-4}$ |

| Produits testés | [$^3$H] PK 11195 $K_{0.5}$ (M) | [$^3$H] RO 54864 $K_{0.5}$ (M) | [$^3$H] RO 151788 $K_{0.5}$ (M) |
|---|---|---|---|
| Exemple 30 | 2,8 $10^{-7}$ | 3,4 $10^{-8}$ | 1,2 $10^{-4}$ |
| Exemple 31 | 1,4 $10^{-6}$ | 2,8 $10^{-6}$ | $10^{-4}$ |
| Exemple 32 | 2,4 $10^{-9}$ | 3,1 $10^{-9}$ | > $10^{-4}$ |

| Produits de référence | [$^3$H] PK 11195 $K_{0.5}$ (M) | [$^3$H] RO 54864 $K_{0.5}$ (M) | [$^3$H] RO 151788 $K_{0.5}$ (M) |
|---|---|---|---|
| Efloxate | 2 $10^{-5}$ | 2 $10^{-4}$ | 2 $10^{-5}$ |
| Dipyridamole | 3 $10^{-7}$ | 2 $10^{-7}$ | 2,8 $10^{-7}$ |
| RO5-4864 | 1 $10^{-7}$ | 9 $10^{-11}$ | 6 $10^{-5}$ |
| PK 11195 | 1 $10^{-9}$ | 2 $10^{-10}$ | 1,5 $10^{-5}$ |
| Diazepam | 4 $10^{-7}$ | 2 $10^{-7}$ | 2,7 $10^{-9}$ |

FIGURE 1

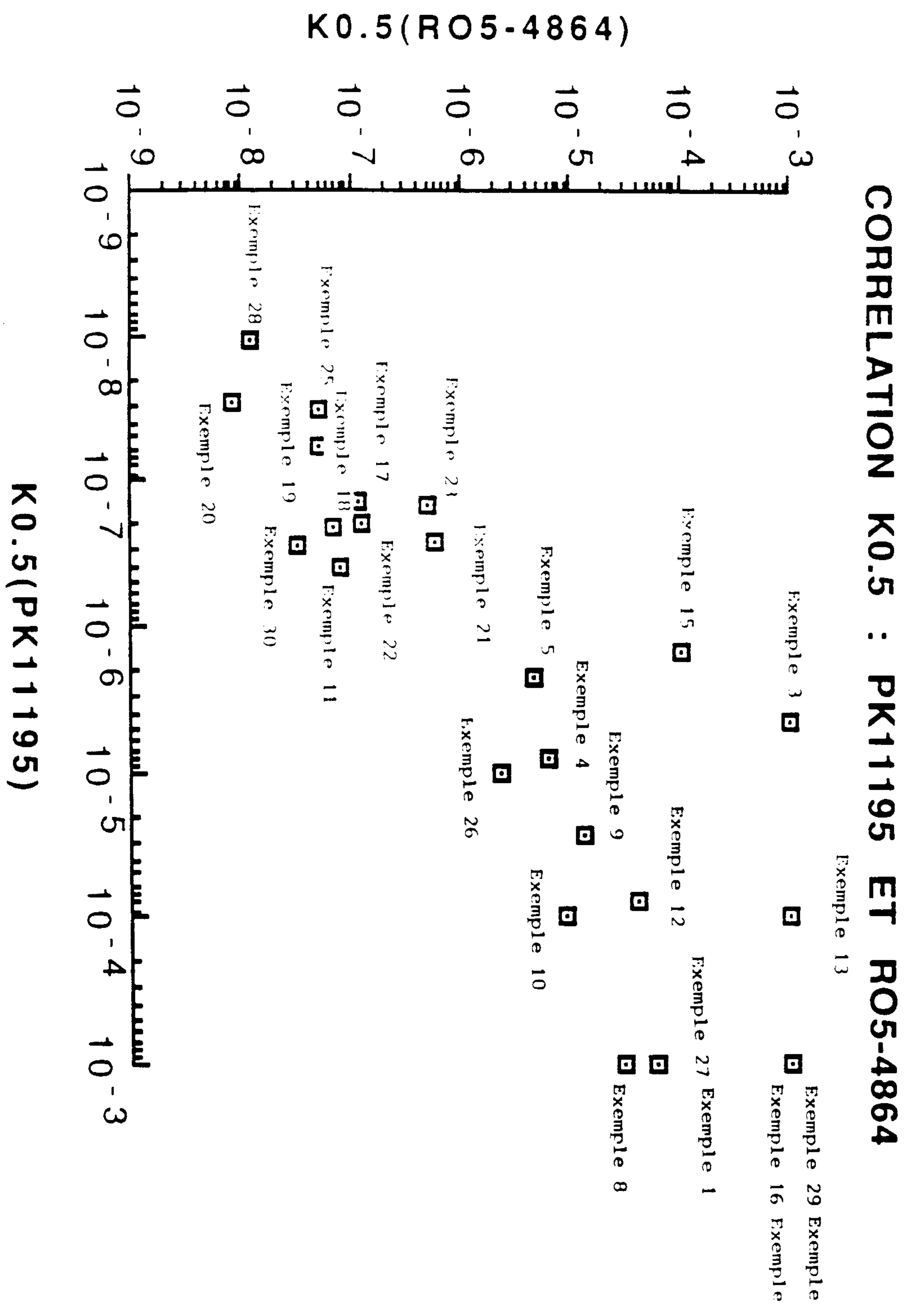
CORRELATION K0.5 : PK11195 ET RO5-4864
K0.5(RO5-4864)
K0.5(PK11195)
10 - 9
10 - 8
10 - 7
10 - 6
10 - 5
10 - 4
10 - 3
Exemple 3
Exemple 13
Exemple 29 Exemple 14
Exemple 16 Exemple 24
Exemple 27 Exemple 1
Exemple 8
Exemple 15
Exemple 12
Exemple 9
Exemple 10
Exemple 5
Exemple 4
Exemple 21
Exemple 26
Exemple 22
Exemple 11
Exemple 17
Exemple 18
Exemple 19
Exemple 30
Exemple 20
Exemple 28

## TABLEAU II

Effet des composés de l'invention sur la tension coronaire induite à la PFG2α

| Produits testés | Nombre d'expériences | $DE_{20}$ (M) | $DE_{50}$ (M) | Intervalle de confiance $[10^{-6}M]$ |
|---|---|---|---|---|
| Exemple 1 | 4 | | | Pas d'effet |
| Exemple 2 | 0 | | | |
| Exemple 3 | 5 | $3,54\ 10^{-5}$ | $7,48\ 10^{-6}$ | |
| Exemple 4 | 5 | $5,74\ 10^{-5}$ | $9,66\ 10^{-6}$ | |
| Exemple 5 | 5 | $1,44\ 10^{-4}$ | $1,89\ 10^{-5}$ | $0,83 < DE50 < 431,9$ |
| Exemple 8 | 4 | | | Pas d'effet |
| Exemple 9 | 4 | | | Pas d'effet |
| Exemple 10 | 7 | $3,33\ 10^{-6}$ | $1,04\ 10^{-6}$ | $0,39 < DE50 < 2,79$ |
| Exemple 11 | 6 | $6,93\ 10^{-6}$ | $1,13\ 10^{-6}$ | $0,28 < DE50 < 4,59$ |
| Exemple 12 | 5 | $5,87\ 10^{-4}$ | $3,19\ 10^{-5}$ | |
| Exemple 13 | 5 | $8,42\ 10^{-5}$ | $7,15\ 10^{-6}$ | |
| Exemple 14 | 6 | $3,86\ 10^{-4}$ | $1,38\ 10^{-5}$ | |
| Exemple 15 | 5 | $4,54\ 10^{-5}$ | $6,12\ 10^{-6}$ | |
| Exemple 16 | 5 | $1,23\ 10^{-5}$ | $3,49\ 10^{-6}$ | |
| Exemple 17 | 5 | $2,22\ 10^{-6}$ | $4,5\ 10^{-7}$ | $0,11 < DE50 < 1,79$ |
| Exemple 18 | 5 | $8,37\ 10^{-6}$ | $9,4\ 10^{-7}$ | $0,165 < DE50 < 5,37$ |
| Exemple 19 | 5 | $2,36\ 10^{-5}$ | $2,36\ 10^{-6}$ | $0,32 < DE50 < 17,63$ |
| Exemple 20 | 5 | $3,46\ 10^{-6}$ | $8,7\ 10^{-7}$ | $0,19 < DE50 < 4,0$ |
| Exemple 21 | 5 | $3,89\ 10^{-6}$ | $1,0\ 10^{-6}$ | $0,28 < DE50 < 3,56$ |
| Exemple 22 | 4 | $3,51\ 10^{-6}$ | $7,3\ 10^{-7}$ | $0,16 < DE50 < 3,39$ |
| Exemple 23 | 5 | $7,4\ 10^{-7}$ | $1,8\ 10^{-7}$ | $0,048 < DE50 < 0,645$ |
| Exemple 24 | 4 | $2,41\ 10^{-5}$ | $9,9\ 10^{-7}$ | $0,067 < DE50 < 14,56$ |
| Exemple 25 | 5 | $\geqslant 10^{-4}$ | $3,7\ 10^{-4}$ | $0,008 < DE50$ |
| Exemple 26 | 5 | $4,53\ 10^{-5}$ | $7,67\ 10^{-6}$ | $1,00 < DE50 < 58,78$ |
| Exemple 27 | 5 | $\geqslant 10^{-4}$ | $\geqslant 10^{-4}$ | |
| Exemple 28 | 5 | $\geqslant 10^{-4}$ | $1,96\ 10^{-4}$ | $0,02 < DE50$ |
| Exemple 29 | 5 | $\geqslant 10^{-4}$ | $\geqslant 10^{-4}$ | |
| Exemple 30 | 5 | $7,39\ 10^{-5}$ | $1,04\ 10^{-5}$ | $0,90 < DE50 < 119,4$ |

| Produits de référence | Nombre d'expériences | $DE_{20}$ (M) | $DE_{50}$ (M) | Intervalle de confiance [$10^{-6}$M] |
|---|---|---|---|---|
| Efloxate | 7 | 3,58 $10^{-5}$ | 3,94 $10^{-6}$ | 0,94 <DE50< 16,54 |
| Dipyri-damole | 6 | 1,61 $10^{-6}$ | 4,46 $10^{-7}$ | 0,2 <DE50<0,99 |
| RO5-4864 | 9 | 9,38 $10^{-6}$ | 1,48 $10^{-6}$ | 0,6 <DE50<3,62 |
| PK 11195 | 7 | 9,13 $10^{-6}$ | 1,66 $10^{-6}$ | 0,63 <DE50<4,35 |
| Diazepam | 7 | 1,64 $10^{-5}$ | / | 0,99 <DE50<7,10 |

**C) Conclusion**

Les résultats précédents montrent que nous sommes en présence de composés qui s'associent aux sites périphériques dits des benzodiazépines et relaxent le muscle lisse coronaire. La sélectivité d'action de ces composés pour le muscle lisse et plus particulièrement le muscle lisse vasculaire conduit à des applications thérapeutiques importantes dans le traitement de la crise angineuse aigüe, le traitement prophylactique de la crise d'angine de poitrine, le traitement post-ischémique, dans l'artérosclérose oblitérante et la vasodilatation périphérique, dans les maladies bronchiques et l'asthme.

**Revendications**

**Revendications pour les Etats contractants suivants : AT, BE, CH, DE, DK, FR, GB, IT, LI, LU, NL, SE**

**1.** Les dérivés d'imidazo [1,2-c] quinazoline de formule générale I :

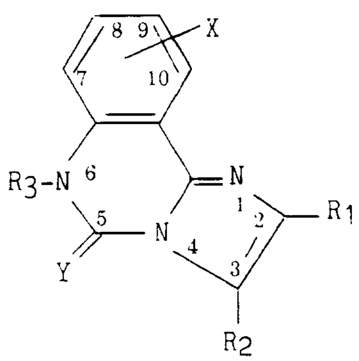

(I)

dans laquelle :

- Y représente un atome d'oxygène ou de soufre ;
- $R_1$ représente :

  a) un radical alkyle ayant de 1 à 6 atomes de carbone en chaine droite ou ramifiée, éventuellement substitué par un radical phényle lui-même éventuellement mono- ou di-substitué par un atome d'halogène ou un radical alkyl ou alkoxy ayant chacun de 1 à 6 atomes de carbone en chaine droite ou ramifiée ;

  b) un radical cycloalkyle mono bi ou tricyclique renfermant de 3 à 10 atomes de carbone ;

  c) un groupe aromatique choisi parmi le groupe formé de :

  - un radical phényle non substitué et les radicaux phényles mono- et di-substitués par un substituant choisi parmi les atomes d'halogène et les radicaux alkyle et alkoxy ayant chacun de 1 à 6 atomes de carbone en chaine droite et ramifiée, et
  - un radical furyle et un radical thiényle, ou

d) un radical acyle choisi parmi les radicaux alkoxycarbonyle, aminocarbonyle, N,N-dialkylaminocarbonyle dans lequel le groupe alkyle renferme de 1 à 6 atomes de carbone en chaine droite ou ramifiée, ou un radical benzoyle éventuellement mono- ou di-substitué par un atome d'halogène ou un radical alkyl ou alkoxy ayant chacun de 1 à 6 atomes de carbone en chaine droite ou ramifiée ;

- $R_2$ représente un atome d'hydrogène ou d'halogène ou un radical alkyle contenant de 1 à 6 atomes de carbone éventuellement substitué par un radical amino alkylamino ou dialkyl amino dans lequel la partie alkyle renferme de 1 à 6 atomes de carbone en chaine droite ou ramifiée ;
- $R_3$ représente :

a) un atome d'hydrogène,

b) un radical alkyle ayant de 1 à 6 atomes de carbone en chaine droite ou ramifiée, éventuellement substitué par un radical phényle, lui-même éventuellement mono- ou poly-substitué par un radical alkyle ou alkoxy ayant chacun de 1 à 6 atomes de carbone en chaine droite ou ramifiée, ou :

c) un radical de formule :

R-CO-A-

dans laquelle :

- A est un radical alkylène en chaîne droite ou ramifiée ayant de 1 à 6 atomes de carbone, et
- R est un radical alkoxy ayant de 1 à 6 atomes de carbone ou un radical amino de formule :

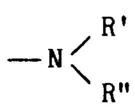

dans laquelle R' et R'', identiques ou différents, représentent chacun un atome d'hydrogène ou un radical alkyle contenant de 1 à 6 atomes de carbone en chaîne droite ou ramifiée éventuellement substitué par un radical hydroxy ou alkoxy ayant de 1 à 6 atomes de carbone, ou R' et R'' forment ensemble avec l'atome d'azote auquel ils sont liés, un radical hétérocyclique hexagonal choisi parmi les radicaux morpholinyle ou N-méthyl pipérazinyle,

d) un radical phényle, éventuellement substitué par un ou plusieurs radicaux alkyle ou alkoxy ayant chacun de 1 à 6 atomes de carbone ou chaine droite ou ramifiée,

- X représente un atome d'hydrogène ou d'halogène,

et la phényl-2 pipéridinocarboxylméthyl-6-oxo-5 dihydro-5,6 imidazo [1,2-c] quinazoline.
et leurs sels physiologiquement tolérables avec des acides appropriés.

**2.** Le dérivé selon la revendication 1 qui est l'éthoxycarbonyl-2 benzyl-6 oxo-5 dihydro-5,6 imidazo [1,2-c] quinazoline.

**3.** Le dérivé selon la revendication 1 qui est la phényl-2 N,N-diéthylaminocarbonylméthyl-6 oxo-5 dihydro-5,6 imidazo [1,2-c] quinazoline.

**4.** Le dérivé selon la revendication 1 qui est la p. méthoxyphényl-2 N,N-diéthylaminocarbonylméthyl-6 oxo-5 dihydro-5,6 imidazo [1,2-c] quinazoline.

**5.** Le dérivé selon la revendication 1 qui est la phényl-2 (N-méthyl N-phénylacétamido)-6 oxo-5 dihydro-5,6 imidazo [1,2-c] quinazoline.

**6.** Le dérivé selon la revendication 1 qui est la phényl-2 N,N-dipropylaminocarbonylméthyl-6 oxo-5 dihydro-5,6 imidazo [1,2-c] quinazoline.

**7.** Le dérivé selon la revendication 1 qui est la m. chlorophényl-2 méthyl-3 N,N-dipropylaminocarbonylméthyl-6 oxo-5 dihydro-5,6 imidazo [1,2-c] quinazoline.

**8.** Le procédé de préparation des dérivés de la revendication 1 caractérisé en ce que :

- l'on fait réagir sur le dérivé de formule générale II :

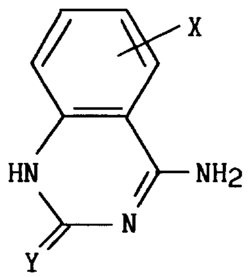

(II)

dans laquelle X et Y ont les significations définies dans la revendication 1, un dérivé halogéné de formule générale III :

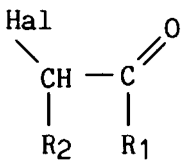

(III)

dans laquelle $R_1$ et $R_2$ ont les significations définies dans la revendication 1 et Hal représente un atome d'halogène, pour obtenir le dérivé de formule générale $I_a$ :

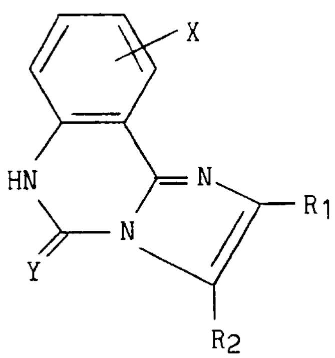

($I_a$)

dans laquelle X, Y, $R_1$ et $R_2$ ont les significations définies dans la revendication 1 ;

- et l'on traite le dérivé $I_a$ ainsi obtenu par une base telle que l'hydrure de sodium, puis par un agent alkylant de formule générale IV :

$R'_3$-Hal (IV)

dans laquelle :

$R'_3$ représente :

a) un radical alkyle ayant de 1 à 5 atomes de carbone en chaîne droite ou ramifiée, éventuellement substitué par un radical phényle lui-même éventuellement mono- ou poly- substitué par un radical alkyle ou alkoxy ayant chacun de 1 à 5 atomes de carbone en chaine droite ou ramifiée, ou

b) un radical de formule :

R-CO-A-

dans laquelle R et A ont les significations définies dans la revendication 1, et

Hal représente un atome d'halogène,

pour obtenir le dérivé de formule générale $I_b$ :

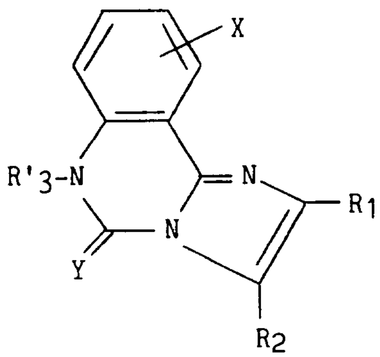

($I_b$)

dans laquelle X, Y, $R_1$, $R_2$ et $R'_3$ ont les significations précédemment définies.

**9.** Le procédé de préparation selon la revendication 8 caractérisé en ce que l'on effectue la réaction des dérivés II et III dans un solvant, à une température comprise entre 25 et 120 ° C.

**10.** Le procédé de préparation selon la revendication 8 caractérisé en ce que l'on traite le dérivé $I_a$ par l'hydrure de sodium à température ambiante, sous atmosphère inerte, dans un solvant dipolaire aprotique.

**11.** Le procédé de préparation des dérivés de la revendication 1 répondant à la formule générale $I_c$ :

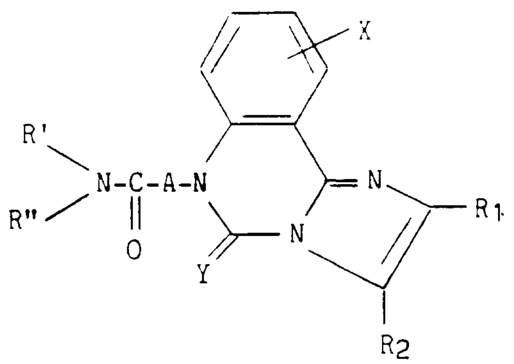

($I_c$)

dans laquelle X, Y, $R_1$, $R_2$, A, R' et R" ont les significations définies dans la revendication 1 caractérisé en ce que :

- l'on hydrolyse en milieu HHal/$CH_3COOH$ les dérivés de formule générale $I_d$ :

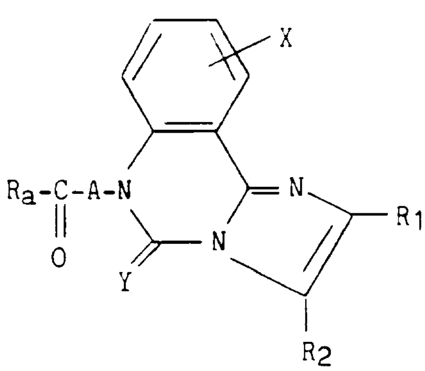

(Id)

dans laquelle X, Y, $R_1$, $R_2$ et A ont les significations définies dans la revendication 1 et $R_a$ représente un radical alkoxy ayant de 1 à 6 atomes de carbone, pour donner les acides

correspondants de formule générale V :

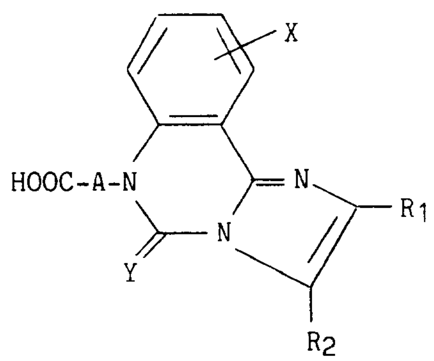

(V)

dans laquelle X, Y, $R_1$, $R_2$ et A ont les significations définies dans la revendication 1, lesquels acides, traités par le chlorure d'oxalyle dans le tétrahydrofuranne à 50-60 °C, donnent les chlorures d'acide correspondants qui, sans étape de purification, sont traités par une amine de formule :

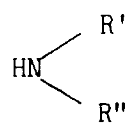

dans laquelle R' et R'' ont les significations définies dans la revendication 1.

**12.** Les compositions pharmaceutiques contenant comme principe actif un dérivé selon les revendications 1 à 7 avec des excipients pharmaceutiques appropriés.

**13.** Les compositions pharmaceutiques selon la revendication 12, présentées sous une forme convenant notamment pour le traitement de la crise angineuse aiguë, le traitement prophylactique de la crise d'angine de poitrine et de l'ischémie, le traitement de l'hypertension, de l'artériosclérose et d'autres maladies hyperprolifératives, et le traitement de l'asthme, de l'anxiété, de la dépression et des maladies d'origine immunologique.

**Revendications pour les Etats contractants suivants : ES, GR**

**1.** Procédé de préparation de dérivés d'imidazo [1,2-c] quinazoline de formule générale I :

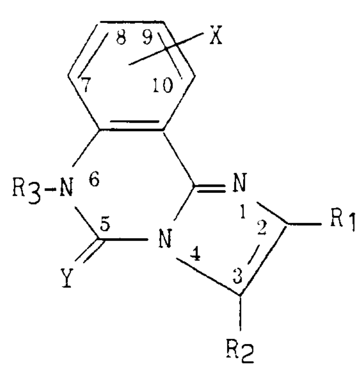

(I)

dans laquelle :

- Y représente un atome d'oxygène ou de soufre ;
- $R_1$ représente :

a) un radical alkyle ayant de 1 à 6 atomes de carbone en chaine droite ou ramifiée, éventuellement substitué par un radical phényle lui-même éventuellement mono- ou di-substitué par un atome d'halogène ou un radical alkyl ou alkoxy ayant chacun de 1 à 6 atomes de carbone en chaine droite ou ramifiée ;

b) un radical cycloalkyle mono bi ou tricyclique renfermant de 3 à 10 atomes de carbone ;

c) un groupe aromatique choisi parmi le groupe formé de :

- un radical phényle non substitué et les radicaux phényles mono- et di-substitués par un substituant choisi parmi les atomes d'halogène et les radicaux alkyle et alkoxy ayant chacun de 1 à 6 atomes de carbone en chaine droite et ramifiée, et
- un radical furyle et un radical thiényle, ou

d) un radical acyle choisi parmi les radicaux alkoxycarbonyle, aminocarbonyle, N,N-dialkylaminocarbonyle dans lequel le groupe alkyle renferme de 1 à 6 atomes de carbone en chaine droite ou ramifiée, ou un radical benzoyle éventuellement mono- ou di-substitué par un atome d'halogène ou un radical alkyl ou alkoxy ayant chacun de 1 à 6 atomes de carbone en chaine droite ou ramifiée ;

- $R_2$ représente un atome d'hydrogène ou d'halogène ou un radical alkyle contenant de 1 à 6 atomes de carbone éventuellement substitué par un radical amino alkylamino ou dialkyl amino dans lequel la partie alkyle renferme de 1 à 6 atomes de carbone en chaine droite ou ramifiée ;
- $R_3$ représente :

a) un atome d'hydrogène,

b) un radical alkyle ayant de 1 à 6 atomes de carbone en chaine droite ou ramifiée, éventuellement substitué par un radical phényle, lui-même éventuellement mono- ou poly-substitué par un radical alkyle ou alkoxy ayant chacun de 1 à 6 atomes de carbone en chaine droite ou ramifiée, ou :

c) un radical de formule :

R-CO-A-

dans laquelle :

- A est un radical alkylène en chaîne droite ou ramifiée ayant de 1 à 6 atomes de carbone, et
- R est un radical alkoxy ayant de 1 à 6 atomes de carbone ou un radical amino de formule :

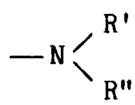

dans laquelle R' et R'', identiques ou différents, représentent chacun un atome d'hydrogène ou un radical alkyle contenant de 1 à 6 atomes de carbone en chaîne droite ou ramifiée éventuellement substitué par un radical hydroxy ou alkoxy ayant de 1 à 6 atomes de carbone, ou R' et R'' forment ensemble avec l'atome d'azote auquel ils sont liés, un radical hétérocyclique hexagonal choisi parmi les radicaux morpholinyle ou N-méthyl pipérazinyle,

d) un radical phényle, éventuellement substitué par un ou plusieurs radicaux alkyle ou alkoxy ayant chacun de 1 à 6 atomes de carbone ou chaine droite ou ramifiée,

- X représente un atome d'hydrogène ou d'halogène,

et la phényl-2 pipéridinocarboxylméthyl-6 oxo-5 dihydro-5,6 imidazo [1,2-c] quinazoline,

- et de leurs sels physiologiquement tolérables avec des acides appropriés,

caractérisé en ce que :

- l'on fait réagir sur le dérivé de formule générale II :

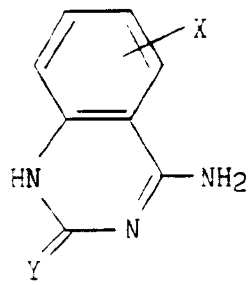

(II)

dans laquelle X et Y ont les significations définies ci-dessus , un dérivé halogéné de formule générale III :

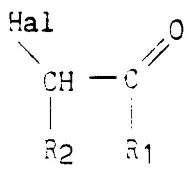

(III)

dans laquelle $R_1$ et $R_2$ ont les significations définies ci-dessus et Hal représente un atome d'halogène, pour obtenir le dérivé de formule générale $I_a$ :

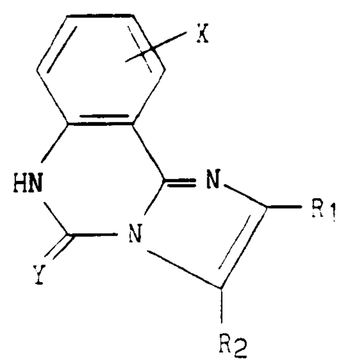

($I_a$)

dans laquelle X, Y, $R_1$ et $R_2$ ont les significations définies ci-dessus ;

- et l'on traite le dérivé $I_a$ ainsi obtenu par une base telle que l'hydrure de sodium, puis par un agent alkylant de formule générale IV :

R'$_3$-Hal (IV)

dans laquelle :
R'$_3$ représente :
a) un radical alkyle ayant de 1 à 5 atomes de carbone en chaîne droite ou ramifiée, éventuellement substitué par un radical phényle lui-même éventuellement mono- ou poly- substitué par un radical alkyle ou alkoxy ayant chacun de 1 à 5 atomes de carbone en chaine droite ou ramifiée, ou
b) un radical de formule :

R-CO-A-

dans laquelle R et A ont les significations définies ci- dessus , et
Hal représente un atome d'halogène,
pour obtenir le dérivé de formule générale $I_b$ :

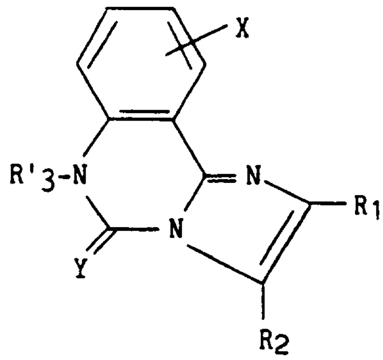

(Ib)

dans laquelle X, Y, $R_1$, $R_2$ et $R'_3$ ont les significations précédemment définies.

2. Le procédé de préparation selon la revendication 1 caractérisé en ce que l'on effectue la réaction des dérivés II et III dans un solvant, à une température comprise entre 25 et 120 ° C.

3. Le procédé de préparation selon la revendication 1 caractérisé en ce que l'on traite le dérivé $I_a$ par l'hydrure de sodium à température ambiante, sous atmosphère inerte, dans un solvant dipolaire aprotique.

4. Le procédé de préparation selon la revendication 1 des dérivés de la revendication 1 répondant à la formule générale $I_c$ :

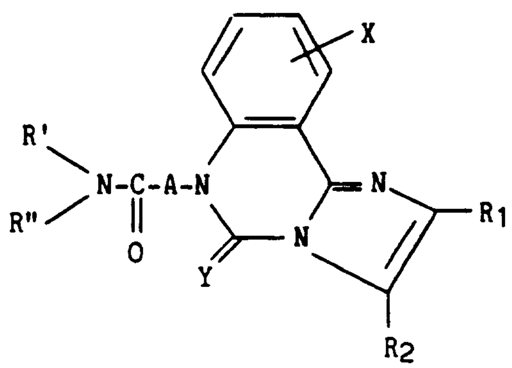

(Ic)

dans laquelle X, Y, $R_1$, $R_2$, A, R' et R'' ont les significations définies dans la revendication 1 caractérisé en ce que :

- l'on hydrolyse en milieu HHal/$CH_3COOH$ les dérivés de formule générale $I_d$ :

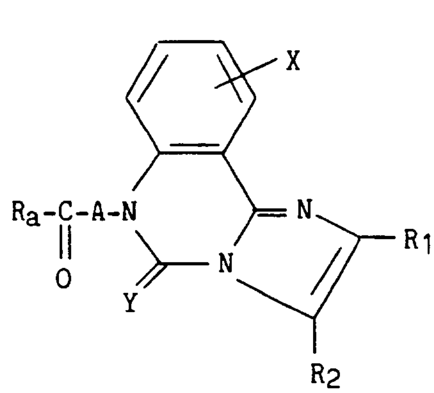

(Id)

dans laquelle X, Y, $R_1$, $R_2$ et A ont les significations définies dans la revendication 1 et $R_a$ représente un radical alkoxy ayant de 1 à 6 atomes de carbone, pour donner les acides

correspondants de formule générale V :

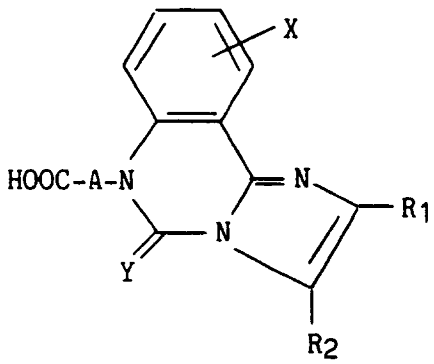

(V)

dans laquelle X, Y, $R_1$, $R_2$ et A ont les significations définies dans la revendication 1,
lesquels acides, traités par le chlorure d'oxalyle dans le tétrahydrofuranne a 50-60 °C, donnent les chlorures d'acide correspondants qui, sans étape de purification, sont traités par une amine de formule :

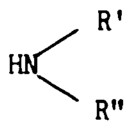

dans laquelle R' et R'' ont les significations définies dans la revendication 1.

**5.** Procédé de préparation selon la revendication 1 de composé selon la revendication 1 qui est l'éthoxycarbonyl-2 benzyl-6 oxo-5 dihydro-5,6 imidazo [1,2-c] quinazoline et ses sels.

**6.** Procédé de préparation selon la revendication 1 de composé selon la revendication 1 qui est la phényl-2 N,N-diéthylaminocarbonylméthyl-6 oxo-5 dihydro-5,6 imidazo [1,2-c] quinazoline, et ses sels.

**7.** Procédé de préparation selon la revendication 1 de composé selon la revendication 1 qui est la p. méthoxyphényl-2 N,N-diéthylaminocarbonylméthyl-6oxo-5 dihydro-5,6 imidazo [1,2-c] quinazoline et ses sels.

**8.** Procédé de préparation selon la revendication 1 de composé selon la revendication 1 qui est la phényl-2 (N-méthyl N-phénylacétamido)-6 oxo-5 dihydro-5,6 imidazo [1,2-c] quinazoline et ses sels.

**9.** Procédé de préparation selon la revendication 1 de composé selon la revendication 1 qui est la phényl-2 N,N-dipropylaminocarbonylméthyl-6 oxo-5 dihydro-5,6 imidazo [1,2-c] quinazoline et ses sels.

**10.** Procédé de préparation selon la revendication 1 de composé selon la revendication 1 qui est la m. chlorophényl-2 méthyl-3 N,N-dipropylaminocarbonylméthyl-6 oxo-5 dihydro-5,6 imidazo [1,2-c] quinazoline et ses sels.

**11.** Procédé de préparation des compositions pharmaceutiques contenant comme principe actif un dérivé de formule générale 1 selon la revendication 1 avec des excipients pharmaceutiques appropriés.

**12.** Les compositions pharmaceutiques selon la revendication 11, présentées sous une forme convenant notamment pour le traitement de la crise angineuse aiguë, le traitement prophylactique de la crise d'angine de poitrine et de l'ischémie, le traitement de l'hypertension, de l'artériosclérose et d'autres maladies hyperprolifératives, et le traitement de l'asthme, de l'anxiété, de la dépression et des maladies d'origine immunologique.

## Claims

**Claims for the following Contracting States : AT, BE, CH, DE, DK, FR, GB, IT, LI, LU, NL, SE**

1. Imidazo[1,2-c]quinazoline compounds of the general formula I:

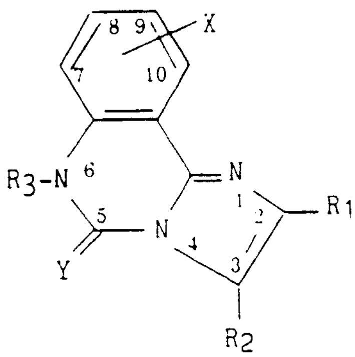

(I)

in which:

- Y represents an oxygen atom or a sulphur atom;
- $R_1$ represents:

  a) an alkyl radical having from 1 to 6 carbon atoms in a straight or branched chain that is optionally substituted by a phenyl radical that is itself optionally mono- or di-substituted by a halogen atom or an alkyl or alkoxy radical each having from 1 to 6 carbon atoms in a straight or branched chain;

  b) a mono-, bi- or tri-cyclic cycloalkyl radical containing from 3 to 10 carbon atoms;

  c) an aromatic group selected from the group consisting of:

    - an unsubstituted phenyl radical and phenyl radicals mono- and di-substituted by a substituent selected from halogen atoms and alkyl and alkoxy radicals each having from 1 to 6 carbon atoms in a straight and branched chain, and
    - a furyl radical and a thienyl radical; or

  d) an acyl radical selected from the radicals alkoxycarbonyl, aminocarbonyl, N,N-dialkylaminocarbonyl in which the alkyl group contains from 1 to 6 carbon atoms in a straight or branched chain, or a benzoyl radical that is optionally mono- or di-substituted by a halogen atom or an alkyl or alkoxy radical each having from 1 to 6 carbon atoms in a straight or branched chain;
- $R_2$ represents a hydrogen atom or a halogen atom or an alkyl radical containing from 1 to 6 carbon atoms that is optionally substituted by an amino, alkylamino or dialkylamino radical in which the alkyl moiety contains from 1 to 6 carbon atoms in a straight or branched chain;
- $R_3$ represents:

  a) a hydrogen atom;

  b) an alkyl radical having from 1 to 6 carbon atoms in a straight or branched chain that is optionally substituted by a phenyl radical that is itself optionally mono- or poly-substituted by an alkyl or alkoxy radical each having from 1 to 6 carbon atoms in a straight or branched chain; or

  c) a radical of the formula:

  R-CO-A-

  in which:

  - A is a straight- or branched-chained alkylene radical having from 1 to 6 carbon atoms, and
  - R is an alkoxy radical having from 1 to 6 carbon atoms, or an amino radical of the formula

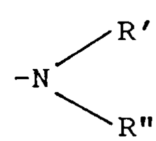

in which each of R' and R'', which may be the same or different, represents a hydrogen atom, or an alkyl radical containing from 1 to 6 carbon atoms in a straight or branched chain that is optionally substituted by a hydroxy or alkoxy radical having from 1 to 6 carbon atoms, or R' and R'', together with the nitrogen atom to which they are bonded, form a heterocyclic radical having 6 ring members and selected from the radicals morpholinyl and N-methylpiperazinyl; or

d) a phenyl radical optionally substituted by one or more alkyl or alkoxy radicals each having from 1 to 6 carbon atoms in a straight or branched chain; and

- X represents a hydrogen atom or a halogen atom;

and 2-phenyl-6-piperidinocarboxymethyl-5-oxo-5,6-dihydroimidazo[1,2-c]quinazoline, and their physiologically tolerable salts with suitable acids.

**2.** Compound according to claim 1 which is 2-ethoxycarbonyl-6-benzyl-5-oxo-5,6-dihydroimidazo[1,2-c]-quinazoline.

**3.** Compound according to claim 1 which is 2-phenyl-6-(N,N-diethylaminocarbonylmethyl)-5-oxo-5,6-dihydroimidazo[1,2-c]quinazoline.

**4.** Compound according to claim 1 which is 2-(p-methoxyphenyl)-6-(N,N-diethylaminocarbonylmethyl)-5-oxo-5,6-dihydroimidazo[1,2-c]quinazoline.

**5.** Compound according to claim 1 which is 2-phenyl-6-(N-methyl-N-phenylacetamido)-5-oxo-5,6-dihydroimidazo[1,2-c]quinazoline.

**6.** Compound according to claim 1 which is 2-phenyl-6-(N,N-dipropylaminocarbonylmethyl)-5-oxo-5,6-dihydroimidazo[1,2-c]quinazoline.

**7.** Compound according to claim 1 which is 2-(m-chlorophenyl)-3-methyl-6-(N,N-dipropylaminocarbonylmethyl)-5-oxo-5,6-dihydroimidazo[1,2-c]quinazoline.

**8.** Process for the preparation of the compounds of claim 1, characterised in that:

- the compound of the general formula II:

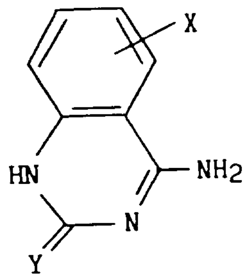

(II),

in which X and Y have the meanings defined in claim 1, is reacted with a halogenated compound of the general formula III:

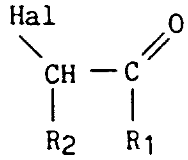

(III),

in which $R_1$ and $R_2$ have the meanings defined in claim 1 and Hal represents a halogen atom, to give the compound of the general formula Ia:

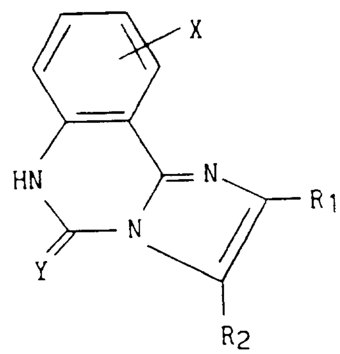

(Ia),

in which X, Y, $R_1$ and $R_2$ have the meanings defined in claim 1;

- and the compound Ia so obtained is treated with a base, such as sodium hydride, and then with an alkylating agent of the general formula IV:

$R'_3$-Hal (IV),

in which:
$R'_3$ represents:
a) an alkyl radical having from 1 to 5 carbon atoms in a straight or branched chain that is optionally substituted by a phenyl radical that is itself optionally mono- or poly-substituted by an alkyl or alkoxy radical each having from 1 to 5 carbon atoms in a straight or branched chain, or
b) a radical of the formula:

R-CO-A-

in which R and A have the meanings defined in claim 1; and
Hal represents a halogen atom,
to give the compound of the general formula Ib:

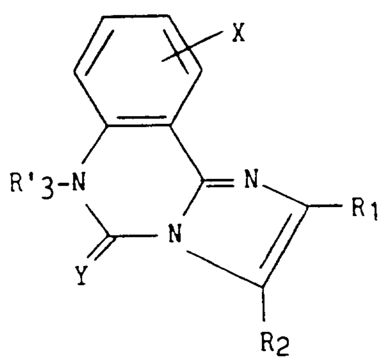

(Ib),

in which X, Y, $R_1$, $R_2$ and $R'_3$ have the meanings defined above.

**9.** Preparation process according to claim 8, characterised in that the reaction of compounds II and III is carried out in a solvent, at a temperature of from 25 to 120 °C.

**10.** Preparation process according to claim 8, characterised in that compound Ia is treated with sodium hydride at room temperature, under an inert atmosphere, in a dipolar aprotic solvent.

**11.** Process for the preparation of the compounds of claim 1 corresponding to the general formula Ic:

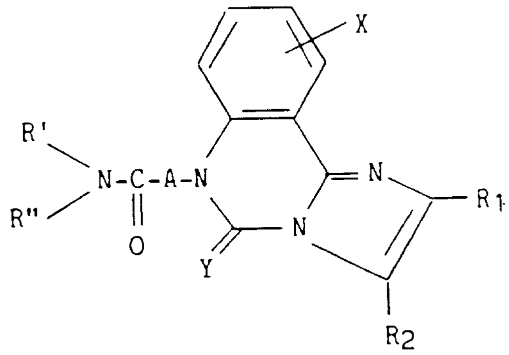

(Ic),

in which X, Y, $R_1$, $R_2$, A, R' and R'' have the meanings defined in claim 1, characterised in that:

- the compounds of the general formula Id:

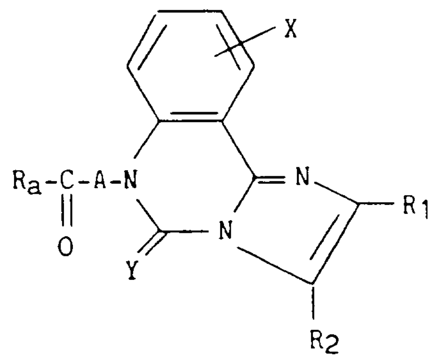

(Id),

in which X, Y, $R_1$, $R_2$ and A have the meanings defined in claim 1 and $R_a$ represents an alkoxy radical having from 1 to 6 carbon atoms, are hydrolysed in a HHal/$CH_3COOH$ medium to give the corresponding acids of the general formula V:

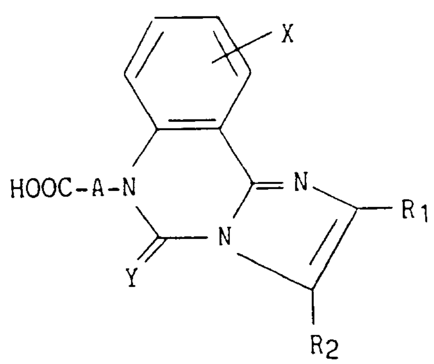

(V),

in which X, Y, $R_1$, $R_2$ and A have the meanings defined in claim 1,

which acids are treated with oxalyl chloride in tetrahydrofuran at from 50 to 60 °C to give the corresponding acid chlorides, which, without a purification step, are treated with an amine of the formula:

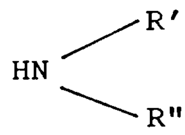

in which R' and R" have the meanings defined in claim 1.

**12.** Pharmaceutical compositions containing as active ingredient a compound according to claims 1 to 7, together with suitable pharmaceutical excipients.

**13.** Pharmaceutical compositions according to claim 12 in a form suitable especially for the treatment of acute anginal attack, for the prophylactic treatment of angina pectoris and ischaemia, for the treatment of hypertension, arteriosclerosis and other hyperproliferative disorders, and for the treatment of asthma, anxiety, depression and disorders of immunological origin.

**Claims for the following Contracting States : ES, GR**

**1.** Process for the preparation of imidazo[1,2-c]quinazoline compounds of the general formula I:

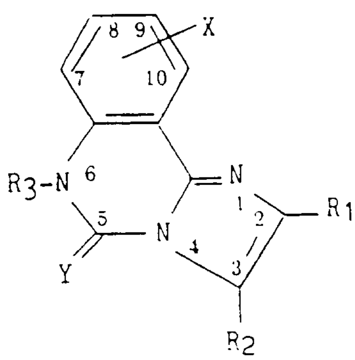

(I)

in which:
- Y represents an oxygen atom or a sulphur atom;
- $R_1$ represents:
  a) an alkyl radical having from 1 to 6 carbon atoms in a straight or branched chain that is optionally substituted by a phenyl radical that is itself optionally mono- or di-substituted by a halogen atom or an alkyl or alkoxy radical each having from 1 to 6 carbon atoms in a straight or branched chain;
  b) a mono-, bi- or tri-cyclic cycloalkyl radical containing from 3 to 10 carbon atoms;
  c) an aromatic group selected from the group consisting of:
    - an unsubstituted phenyl radical and phenyl radicals mono- and di-substituted by a substituent selected from halogen atoms and alkyl and alkoxy radicals each having from 1 to 6 carbon atoms in a straight and branched chain, and
    - a furyl radical and a thienyl radical; or
  d) an acyl radical selected from the radicals alkoxycarbonyl, aminocarbonyl, N,N-dialkylaminocarbonyl in which the alkyl group contains from 1 to 6 carbon atoms in a straight or branched chain, or a benzoyl radical that is optionally mono- or di-substituted by a halogen atom or an alkyl or alkoxy radical each having from 1 to 6 carbon atoms in a straight or branched chain;
- $R_2$ represents a hydrogen atom or a halogen atom or an alkyl radical containing from 1 to 6 carbon atoms that is optionally substituted by an amino, alkylamino or dialkylamino radical in which the alkyl moiety contains from 1 to 6 carbon atoms in a straight or branched chain;
- $R_3$ represents:
  a) a hydrogen atom;
  b) an alkyl radical having from 1 to 6 carbon atoms in a straight or branched chain that is optionally substituted by a phenyl radical that is itself optionally mono- or poly-substituted by an

alkyl or alkoxy radical each having from 1 to 6 carbon atoms in a straight or branched chain; or c) a radical of the formula:

R-CO-A-

in which:

- A is a straight- or branched-chained alkylene radical having from 1 to 6 carbon atoms, and
- R is an alkoxy radical having from 1 to 6 carbon atoms, or an amino radical of the formula

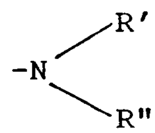

in which each of R' and R", which may be the same or different, represents a hydrogen atom, or an alkyl radical containing from 1 to 6 carbon atoms in a straight or branched chain that is optionally substituted by a hydroxy or alkoxy radical having from 1 to 6 carbon atoms, or R' and R", together with the nitrogen atom to which they are bonded, form a heterocyclic radical having 6 ring members and selected from the radicals morpholinyl and N-methylpiperazinyl; or d) a phenyl radical optionally substituted by one or more alkyl or alkoxy radicals each having from 1 to 6 carbon atoms in a straight or branched chain; and

- X represents a hydrogen atom or a halogen atom; and 2-phenyl-6-piperidinocarboxymethyl-5-oxo-5,6-dihydroimidazo[1,2-c]quinazoline,

and their physiologically tolerable salts with suitable acids,
characterised in that:

- the compound of the general formula II:

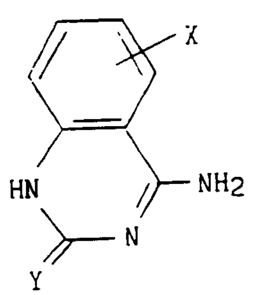

(II),

in which X and Y have the meanings defined above,is is reacted with a halogenated compound of the general formula III:

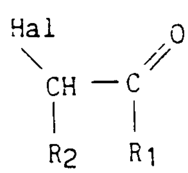

(III),

in which $R_1$ and $R_2$ have the meanings defined above and Hal represents a halogen atom, to give the compound of the general formula Ia:

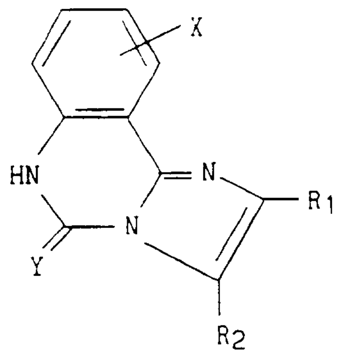

(Ia),

in which X, Y, $R_1$ and $R_2$ have the meanings defined above ;

- and the compound Ia so obtained is treated with a base, such as sodium hydride, and then with an alkylating agent of the general formula IV:

R'$_3$-Hal (IV),

in which:
R'$_3$ represents:
a) an alkyl radical having from 1 to 5 carbon atoms in a straight or branched chain that is optionally substituted by a phenyl radical that is itself optionally mono- or poly-substituted by an alkyl or alkoxy radical each having from 1 to 5 carbon atoms in a straight or branched chain, or
b) a radical of the formula:

R-CO-A-

in which R and A have the meanings defined in claim 1; and
Hal represents a halogen atom,
to give the compound of the general formula Ib:

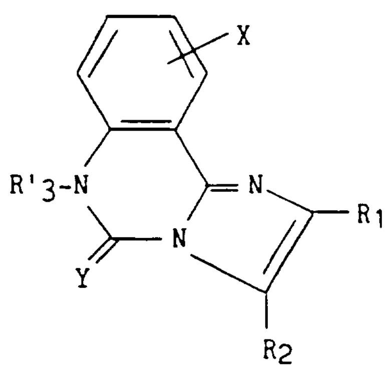

(Ib),

in which X, Y, $R_1$, $R_2$ and R'$_3$ have the meanings defined above.

2. Preparation process according to claim 1, characterised in that the reaction of compounds II and III is carried out in a solvent, at a temperature of from 25 to 120 °C.

3. Preparation process according to claim 1, characterised in that compound Ia is treated with sodium hydride at room temperature, under an inert atmosphere, in a dipolar aprotic solvent.

4. Process according to claim 1 for the preparation of the compounds of claim 1 corresponding to the general formula Ic:

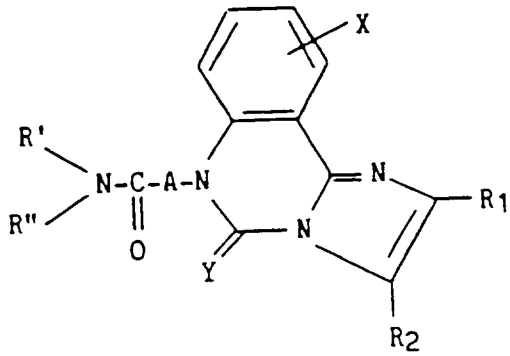

(Ic),

in which X, Y, $R_1$, $R_2$, A, R' and R" have the meanings defined in claim 1, characterised in that:

- the compounds of the general formula Id:

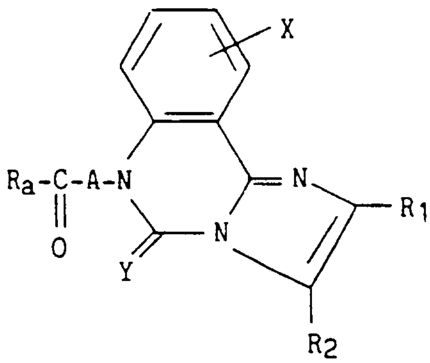

(Id),

in which X, Y, $R_1$, $R_2$ and A have the meanings defined in claim 1 and $R_a$ represents an alkoxy radical having from 1 to 6 carbon atoms, are hydrolysed in a HHal/$CH_3COOH$ medium to give the corresponding acids of the general formula V:

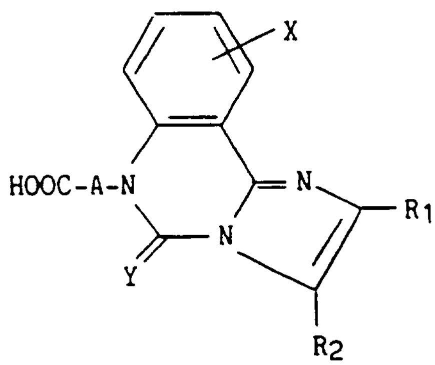

(V),

in which X, Y, $R_1$, $R_2$ and A have the meanings defined in claim 1,

which acids are treated with oxalyl chloride in tetrahydrofuran at from 50 to 60°C to give the corresponding acid chlorides, which, without a purification step, are treated with an amine of the formula:

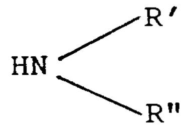

in which R' and R" have the meanings defined in claim 1.

**5.** Process according to claim 1 for the preparation of a compound according to claim 1 which is 2-ethoxycarbonyl-6-benzyl-5-oxo-5,6-dihydroimidazo[1,2-c]quinazoline, and its salts.

**6.** Process according to claim 1 for the preparation of a compound according to claim 1 which is 2-phenyl-6-(N,N-diethylaminocarbonylmethyl)-5-oxo-5,6-dihydroimidazo[1,2-c]quinazoline, and its salts.

**7.** Process according to claim 1 for the preparation of a compound according to claim 1 which is 2-(p-methoxyphenyl)-6-(N,N-diethylaminocarbonylmethyl)-5-oxo-5,6-dihydroimidazo[1,2-c]quinazoline, and its salts.

**8.** Process according to claim 1 for the preparation of a compound according to claim 1 which is 2-phenyl-6-(N-methyl-N-phenylacetamido)-5-oxo-5,6-dihydroimidazo[1,2-c]quinazoline, and its salts.

**9.** Process according to claim 1 for the preparation of a compound according to claim 1 which is 2-phenyl-6-(N,N-dipropylaminocarbonylmethyl)-5-oxo-5,6-dihydroimidazo[1,2-c]quinazoline, and its salts.

**10.** Process according to claim 1 for the preparation of a compound according to claim 1 which is 2-(m-chlorophenyl)-3-methyl-6-(N,N-dipropylaminocarbonylmethyl)-5-oxo-5,6-dihydroimidazo[1,2-c]-quinazoline, and its salts.

**11.** Process for the preparation of pharmaceutical compositions containing as active ingredient a compound of the general formula I according to claim 1, together with suitable pharmaceutical excipients.

**12.** Pharmaceutical compositions according to claim 11 in a form suitable especially for the treatment of acute anginal attack, for the prophylactic treatment of angina pectoris and ischaemia, for the treatment of hypertension, arteriosclerosis and other hyperproliferative disorders, and for the treatment of asthma, anxiety, depression and disorders of immunological origin.

**Patentansprüche**

**Patentansprüche für folgende Vertragsstaaten : AT, BE, CH, DE, DK, FR, GB, IT, LI, LU, NL, SE**

**1.** Imidazo[1,2-c]chinazolin-Derivate der allgemeinen Formel I:

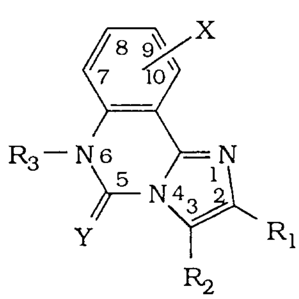

(I)

In der:

- Y ein Sauerstoff- oder Schwefelatom;
- $R_1$:

a) eine Alkylgruppe mit 1 bis 6 Kohlenstoffatomen in gerader oder verzweigter Kette, die gegebenenfalls durch eine Phenylgruppe substituiert ist, die ihrerseits gegebenenfalls durch ein Halogenatom oder eine Alkyl- oder Alkoxygruppe mit jeweils 1 bis 6 Kohlenstoffatomen in gerader oder verzweigter Kette mono- oder disubstituiert ist;
b) eine mono-, bi- oder tricyclische Cycloalkylgruppe mit 3 bis 10 Kohlenstoffatomen;
c) eine aromatische Gruppe ausgewählt aus der Gruppe, welche aus:

- einer nichtsubstituierten Phenylgruppe und mit Substituenten ausgewählt aus Halogenatomen und Alkyl- und Alkoxygruppen mit jeweils 1 bis 6 Kohlenstoffatomen mono- und disubstituierten Phenylgruppen und
- einer Furylgruppe und einer Thienylgruppe gebildet ist, oder

d) eine Acylgruppe ausgewählt aus Alkoxycarbonyl-, Aminocarbonyl-, N,N-Dialkylaminocarbonylgruppen, worin die Alkylgruppe 1 bis 6 Kohlenstoffatome in gerader oder verzweigter Kette umfaßt, oder einer Benzoylgruppe, die gegebenenfalls durch ein Halogenatom oder eine Alkyl- oder Alkoxygruppe mit jeweils 1 bis 6 Kohlenstoffatomen in gerader oder verzweigter Kette mono- oder disubstituiert ist;

- $R_2$ ein Wasserstoffatom, ein Halogenatom oder eine Alkylgruppe, die 1 bis 6 Kohlenstoffatome aufweist und gegebenenfalls durch eine Amino-, Alkylamino- oder Dialkylamino-gruppe substituiert ist, worin der Alkylteil 1 bis 6 Kohlenstoffatome in gerader oder verzweigter Kette umfaßt;
- $R_3$:

a) ein Wasserstoffatom,
b) eine Alkylgruppe mit 1 bis 6 Kohlenstoffatomen in gerader oder verzweigter Kette, die gegebenenfalls durch eine Phenylgruppe substituiert ist, welche ihrerseits gegebenenfalls durch eine Alkyl- oder Alkoxygruppe mit jeweils 1 bis 6 Kohlenstoffatomen in gerader oder verzweigter Kette mono- oder polysubstituiert ist, oder:
c) eine Gruppe der Formel:

R - CO - A -

in der:

- A eine Alkylengruppe mit 1 bis 6 Kohlenstoffatomen in gerader oder verzweigter Kette und
- R eine Alkoxygruppe mit 1 bis 6 Kohlenstoffatomen oder eine Aminogruppe der Formel:

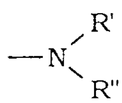

in der R' und R'', die gleichartig oder verschieden sein können, jeweils ein Wasserstoffatom oder eine Alkylgruppe mit 1 bis 6 Kohlenstoffatomen in gerader oder verzweigter Kette enthält und gegebenenfalls durch eine Hydroxyl- oder Alkoxygruppe mit 1 bis 6 Kohlenstoffatomen substituiert ist, darstellen oder R' und R'' gemeinsam mit dem Stickstoffatom, an das sie gebunden sind, eine hexagonale heterocyclische Gruppe ausgewählt aus Morpholinyl- oder N-Methyl-piperazinylgruppen bilden, darstellen,
d) eine Phenylgruppe, die gegebenenfalls durch eine oder mehrere Alkyl- oder Alkoxygruppen mitjeweils 1 bis 6 Kohlenstoffatomen in gerader oder verzweigter Kette substituiert ist, und

- X ein Wasserstoffatom oder ein Halogenatom bedeuten und

2-Phenyl-6-piperidinocarboxymethyl-5-oxo-5,6-dihydro-imidazo[1,2-c]chinazolin und deren physiologisch verträgliche Salze mit geeigneten Säuren.

**2.** Derivat nach Anspruch 1, nämlich 2-Ethoxycarbonyl-6-benzyl-5-oxo-5,6-dihydro-imidazo[1,2-c]-chinazolin.

**3.** Derivat nach Anspruch 1, nämlich 2-Phenyl-6-N,N-diethylaminocarbonylmethyl-5-oxo-5,6-dihydro-imidazo[1,2-c]chinazolin.

**4.** Derivat nach Anspruch 1, nämlich 2-p-Methoxyphenyl-6-N,N-diethylaminocarbonylmethyl-5-oxo-5,6-dihydro-imidazo[1,2c]chinazolin.

**5.** Derivat nach Anspruch 1, nämlich 2-Phenyl-6-(N-methyl-N-phenylacetamido)-5-oxo-5,6-dihydro-imidazo[1,2-c]chinazolin.

**6.** Derivat nach Anspruch 1, nämlich 2-Phenyl-6-N,N-dipropylaminocarbonylmethyl-5-oxo-5,6-dihydro-imidazo[1,2-c]chinazolin.

**7.** Derivat nach Anspruch 1, nämlich 2-m-Chlorphenyl-3-methyl-6-N,N-tripropylaminocarbonylmethyl-5-oxo-5,6-dihydro-imidazo[1,2-c]chinazolin.

**8.** Verfahren zur Herstellung der Derivate nach Anspruch 1, **dadurch gekennzeichnet,** daß man:

- auf das Derivat der allgemeinen Formel II:

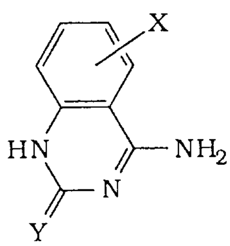

(II)

in der X und Y die in Anspruch 1 angegebenen Bedeutungen besitzen, ein Halogenderivat der allgemeinen Formel III:

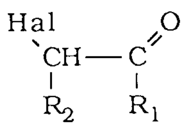

(III)

in der $R_1$ und $R_2$ die in Anspruch 1 angegebenen Bedeutungen besitzen und Hal ein Halogenatom darstellt, einwirken läßt zur Bildung des Derivats der allgemeinen Formel $I_a$:

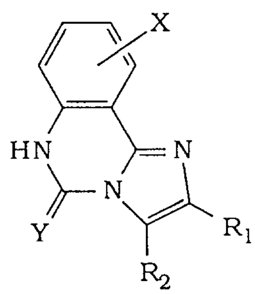

($I_a$)

in der X, Y, $R_1$ und $R_2$ die in Anspruch 1 angegebenen Bedeutungen besitzen;

- und das in dieser Weise erhaltene Derivat $I_a$ mit einer Base, wie Natriumhydrid, und dann mit einem Alkylierungsmittel der allgemeinen Formel IV:

$R'_3$ - Hal (IV)

in der:

$R'_3$:

a) eine Alkylgruppe mit 1 bis 5 Kohlenstoffatomen in gerader oder verzweigter Kette, die gegebenenfalls durch eine Phenylgruppe substituiert ist, welche ihrerseits gegebenenfalls durch eine Alkyl- oder Alkoxygruppe mit jeweils 1 bis 5 Kohlenstoffatomen in gerader oder verzweigter Kette mono- oder polysubstituiert ist, oder

b) eine Gruppe der Formel:

R - CO - A -

in der R und A die in Anspruch 1 angegebenen Bedeutungen besitzen, und Hal ein Halogenatom bedeuten,

behandelt zur Bildung des Derivats der allgemeinen Formel $I_b$:

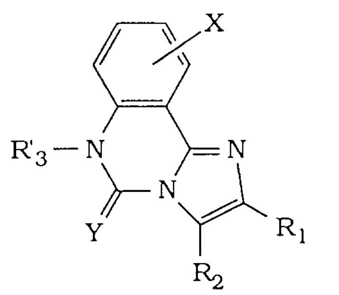

$(I_b)$

in der X, Y, $R_1$, $R_2$ und $R'_3$ die oben angegebenen Bedeutungen besitzen.

**9.** Verfahren nach Anspruch 8, **dadurch gekennzeichnet,** daß man die Umsetzung der Derivate II und III in einem Lösungsmittel bei einer Temperatur zwischen 25 und 120 °C durchführt.

**10.** Verfahren nach Anspruch 9, **dadurch gekennzeichnet,** daß man das Derivat $I_a$ mit Natriumhydrid bei Raumtemperatur unter inerter Atmosphäre und in einem aprotischen dipolaren Lösungsmittel behandelt.

**11.** Verfahren zur Herstellung der Derivate nach Anspruch 1, die der allgemeinen Formel $I_c$ entsprechen:

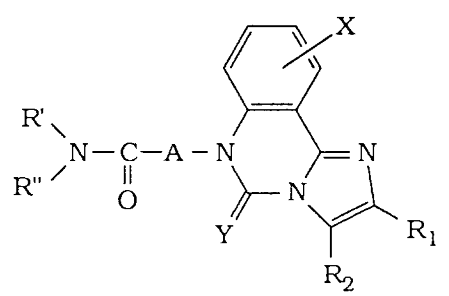

$(I_c)$

in der X, Y, $R_1$, $R_2$, A, R' und R'' die in Anspruch 1 angegebenen Bedeutungen besitzen,
**dadurch gekennzeichnet,** daß man:

- die Derivate der allgemeinen Formel $I_d$:

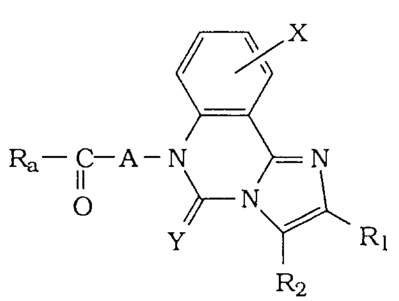

$(I_d)$

in der X, Y, $R_1$, $R_2$ und A die in Anspruch 1 angegebenen Bedeutungen besitzen und $R_a$ eine Alkoxygruppe mit 1 bis 6 Kohlenstoffatomen darstellt, in einem $HHal/CH_3COOH$-Medium hydroly-

siert zur Bildung der entsprechenden Säuren der allgemeinen Formel V:

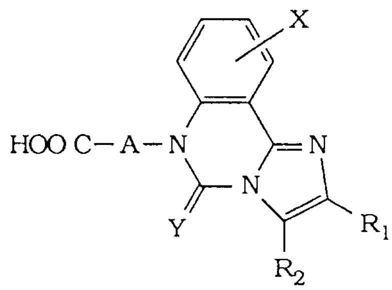

(V)

in der X, Y, $R_1$, $R_2$ und A die in Anspruch 1 angegebenen Bedeutungen besitzen, welche Säuren bei der Behandlung mit Oxalylchlorid in Tetrahydrofuran bei 50-60 °C die entsprechenden Säurechloride ergeben, weiche ohne weitere Reinigung mit einem Amin der Formel:

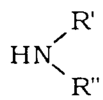

worin R' und R'' die in Anspruch 1 angegebenen Bedeutungen besitzen, behandelt werden.

**12.** Pharmazeutische Zubereitungen enthaltend als Wirkstoff ein Derivat nach den Ansprüchen 1 bis 7 zusammen mit geeigneten pharmazeutischen Trägermaterialien.

**13.** Pharmazeutische Zubereitungen nach Anspruch 12 in einer Form, die insbesondere zur Behandlung von akuten Anginakrisen, der prophylaktischen Behandlung der Krise von Angina pectoris und Ischämie, der Behandlung der Hypertension, der Arteriosklerose und von anderen hyperproliferativen Erkrankungen und zur Behandlung von Asthma, der Angst, der Depression und Krankheiten immunologischen Ursprungs geeignet ist.

**Patentansprüche für folgende Vertragsstaaten : ES, GR**

**1.** Verfahren zur Herstellung von Imidazo[1,2-c]chinazolin-Derivaten der allgemeinen Formel I:

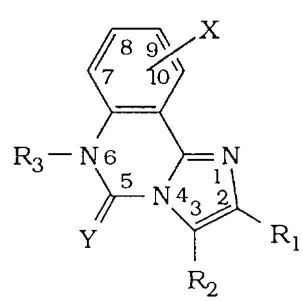

(I)

in der:
- Y ein Sauerstoff- oder Schwefelatom;
- $R_1$:

a) eine Alkylgruppe mit 1 bis 6 Kohlenstoffatomen in gerader oder verzweigter Kette, die gegebenenfalls durch eine Phenylgruppe substituiert ist, die ihrerseits gegebenenfalls durch ein Halogenatom oder eine Alkyl- oder Alkoxygruppe mit jeweils 1 bis 6 Kohlenstoffatomen in gerader oder verzweigter Kette mono- oder disubstituiert ist:

b) eine mono-, bi- oder tricyclische Cycloalkylgruppe mit 3 bis 10 Kohlenstoffatomen;
c) eine aromatische Gruppe ausgewählt aus der Gruppe, welche aus:
- einer nichtsubstituierten Phenylgruppe und mit Substituenten ausgewählt aus Halogenatomen und Alkyl- und Alkoxygruppen mit jeweils 1 bis 6 Kohlenstoffatomen mono- und disubstituierten Phenylgruppen und
- einer Furylgruppe und einer Thienylgruppe gebildet ist, oder

d) eine Acylgruppe ausgewählt aus Alkoxycarbonyl-, Aminocarbonyl-, N,N-Dialkylaminocarbonylgruppen, worin die Alkylgruppe 1 bis 6 Kohlenstoffatome in gerader oder verzweigter Kette umfaßt, oder einer Benzoylgruppe, die gegebenenfalls durch ein Halogenatom oder eine Alkyl- oder Alkoxygruppe mit jeweils 1 bis 6 Kohlenstoffatomen in gerader oder verzweigter Kette mono- oder disubstituiert ist:

- $R_2$ ein Wasserstoffatom, ein Halogenatom oder eine Alkylgruppe, die 1 bis 6 Kohlenstoffatome aufweist und gegebenenfalls durch eine Amino-, Alkylamino- oder Dialkylamino-gruppe substituiert ist, worin der Alkylteil 1 bis 6 Kohlenstoffatome in gerader oder verzweigter Kette umfaßt;
- $R_3$:

a) ein Wasserstoffatom,
b) eine Alkylgruppe mit 1 bis 6 Kohlenstoffatomen in gerader oder verzweigter Kette, die gegebenenfalls durch eine Phenylgruppe substituiert ist, welche ihrerseits gegebenenfalls durch eine Alkyl- oder Alkoxygruppe mit jeweils 1 bis 6 Kohlenstoffatomen in gerader oder verzweigter Kette mono- oder polysubstituiert ist, oder:
c) eine Gruppe der Formel:

R - CO - A -

in der:
- A eine Alkylengruppe mit 1 bis 6 Kohlenstoffatomen in gerader oder verzweigter Kette und
- R eine Alkoxygruppe mit 1 bis 6 Kohlenstoffatomen oder eine Aminogruppe der Formel:

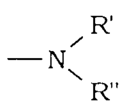

in der R' und R'', die gleichartig oder verschieden sein können, jeweils ein Wasserstoffatom oder eine Alkylgruppe mit 1 bis 6 Kohlenstoffatomen in gerader oder verzweigter Kette enthält und gegebenenfalls durch eine Hydroxyl- oder Alkoxygruppe mit 1 bis 6 Kohlenstoffatomen substituiert ist, darstellen oder R' und R'' gemeinsam mit dem Stickstoffatom, an das sie gebunden sind, eine hexagonale heterocyclische Gruppe ausgewählt aus Morpholinyl- oder N-Methyl-piperazinylgruppen bilden, darstellen,

d) eine Phenylgruppe, die gegebenenfalls durch eine oder mehrere Alkyl- oder Alkoxygruppen mit jeweils 1 bis 6 Kohlenstoffatomen in gerader oder verzweigter Kette substituiert ist, und

- X ein Wasserstoffatom oder ein Halogenatom bedeuten und

2-Phenyl-6-piperidinocarboxymethyl-5-oxo-5,6-dihydro-imidazo[1,2-c]chinazolin und von deren physiologisch verträglichen Salzen mit geeigneten Säuren,

**dadurch gekennzeichnet,** daß man:

- auf das Derivat der allgemeinen Formel II:

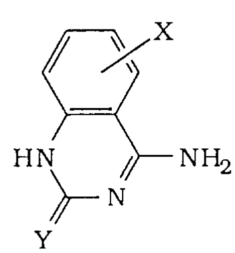

(II)

in der X und Y die oben angegebenen Bedeutungen besitzen, ein Halogenderivat der allgemeinen

Formel III:

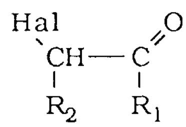 (III)

in der $R_1$ und $R_2$ die oben angegebenen Bedeutungen besitzen und Hal ein Halogenatom darstellt, einwirken läßt zur Bildung des Derivats der allgemeinen Formel $I_a$:

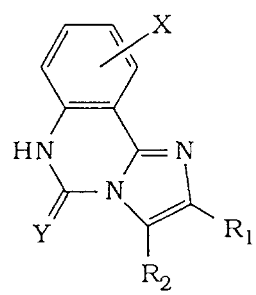 ($I_a$)

in der X, Y, $R_1$ und $R_2$ die oben angegebenen Bedeutungen besitzen;

- und das in dieser Weise erhaltene Derivat $I_a$ mit einer Base, wie Natriumhydrid, und dann mit einem Alkylierungsmittel der allgemeinen Formel IV:

$R'_3$ - Hal (IV)

in der:
$R'_3$:
a) eine Alkylgruppe mit 1 bis 5 Kohlenstoffatomen in gerader oder verzweigter Kette, die gegebenenfalls durch eine Phenylgruppe substituiert ist, welche ihrerseits gegebenenfalls durch eine Alkyl- oder Alkoxygruppe mit jeweils 1 bis 5 Kohlenstoffatomen in gerader oder verzweigter Kette mono- oder polysubstituiert ist, oder
b) eine Gruppe der Formel:

R - CO - A -

in der R und A die oben angegebenen Bedeutungen besitzen, und
Hal ein Halogenatom bedeuten,
behandelt zur Bildung des Derivats der allgemeinen Formel $I_b$:

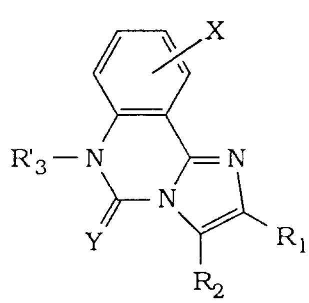 ($I_b$)

in der X, Y, $R_1$, $R_2$ und $R'_3$ die oben angegebenen Bedeutungen besitzen.

2. Verfahren nach Anspruch 1, **dadurch gekennzeichnet,** daß man die Umsetzung der Derivate II und III in einem Lösungsmittel bei einer Temperatur zwischen 25 und 120 °C bewirkt.

**3.** Verfahren nach Anspruch 1, **dadurch gekennzeichnet,** daß man das Derivat $I_a$ bei Raumtemperatur unter inerter Atmosphäre in einem dipolaren aprotischen Lösungsmittel mit Natriumhydrid behandelt.

**4.** Verfahren nach Anspruch 1 zur Herstellung von Derivaten nach Anspruch 1 der allgemeinen Formel $I_c$:

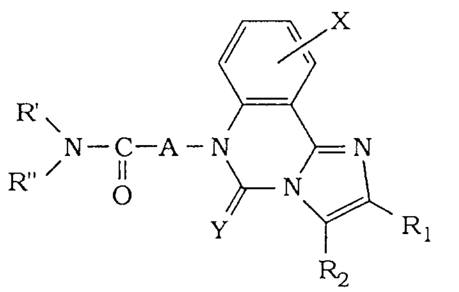

($I_c$)

in der X, Y, $R_1$, $R_2$, A, R' und R'' die in Anspruch 1 angegebenen Bedeutungen besitzen,
**dadurch gekennzeichnet,** daß man:

- die Derivate der allgemeinen Formel $I_d$:

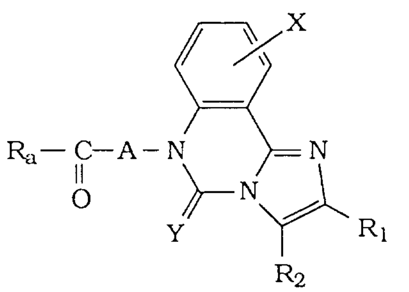

($I_d$)

In der X, Y, $R_1$, $R_2$ und A die in Anspruch 1 angegebenen Bedeutungen besitzen und $R_a$ eine Alkoxygruppe mit 1 bis 6 Kohlenstoffatomen darstellt, in einem $HHal/CH_3COOH$-Medium hydrolysiert zur Bildung der entsprechenden Säuren der allgemeinen Formel V:

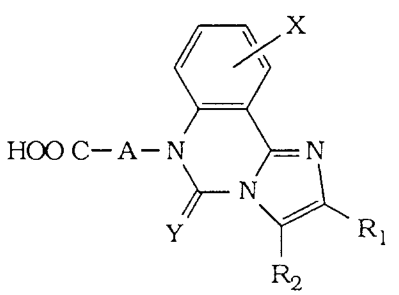

(V)

in der X, Y, $R_1$, $R_2$ und A die in Anspruch 1 angegebenen Bedeutungen besitzen, welche Säuren bei der Behandlung mit Oxalylchlorid in Tetrahydrofuran bei 50-60 °C die entsprechenden Säurechloride ergeben, welche ohne weitere Reinigung mit einem Amin der Formel:

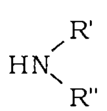

worin R' und R'' die in Anspruch 1 angegebenen Bedeutungen besitzen, behandelt werden.

5. Verfahren nach Anspruch 1 zur Herstellung der Verbindung nach Anspruch 1, nämlich 2-Ethoxycarbonyl-6-benzyl-5-oxo-5,6-dihydro-imidazo[1,2-c]chinazolin und von dessen Salzen.

6. Verfahren nach Anspruch 1 zur Herstellung der Verbindung nach Anspruch 1, nämlich 2-Phenyl-6-N,N-diethylaminocarbonylmethyl-5-oxo-5,6-dihydro-imidazo[1,2-c]chinazolin.

7. Verfahren nach Anspruch 1 zur Herstellung der Verbindung nach Anspruch 1, nämlich 2-p-Methoyphenyl-6-N,N-diethylaminocarbonylmethyl-5-oxo-5,6-dihydro-imidazo[1,2-c]chinazolin.

8. Verfahren nach Anspruch 1 zur Herstellung der Verbindung nach Anspruch 1, nämlich 2-Phenyl-6-(N-methyl-N-phenylacetamido)-5-oxo-5,6-dihydro-imidazol[1,2-c]chinazolin.

9. Verfahren nach Anspruch 1 zur Herstellung der Verbindung nach Anspruch 1, nämlich 2-Phenyl-6-N,N-dipropylaminocarbonylmethyl-5-oxo-5,6-dihydro-imidazo[1,2-c]chinazolin.

10. Verfahren nach Anspruch 1 zur Herstellung der Verbindung nach Anspruch 1, nämlich 2-m-Chlorphenyl-3-methyl-6-N,N-tripropylaminocarbonylmethyl-5-oxo-5,6-dihydro-imidazo[1,2-c]chinazolin.

11. Verfahren zur Herstellung von pharmazeutischen Zubereitungen enthaltend als Wirkstoff ein Derivat der allgemeinen Formel I nach Anspruch 1 zusammen mit geeigneten pharmazeutischen Trägermaterialien.

12. Pharmazeutische Zubereitungen nach Anspruch 11 in einer Form, die insbesondere zur Behandlung von akuten Anginakrisen, der prophylaktischen Behandlung der Krise von Angina pectoris und Ischämie, der Behandlung der Hypertension, der Arteriosklerose und von anderen hyperproliferativen Erkrankungen und zur Behandlung von Asthma, der Angst, der Depression und Krankheiten immunologischen Ursprungs geeignet ist.